# FASCICULE DE BREVET EUROPEEN

(11) **EP 3 204 508 B1**
(45) Date de publication et mention de la délivrance du brevet: **21.08.2024**
(21) Numéro de dépôt: 15788145.9
(22) Date de dépôt: 05.10.2015
(51) Int. Cl.: C12Q 1/04, C12M 1/34, C12Q 1/18, G01N 21/00, G06T 7/00, C12Q 1/20

(54) **MÉTHODE D'ANALYSE ET INTERPRÉTATION AUTOMATISÉE D'UN ANTIBIOGRAMME**
AUTOMATISIERTES VERFAHREN ZUR ANALYSE UND AUSWERTUNG EINES ANTIMIKROBIELLEN SUSZEPTIBILITÄTSTESTS
AUTOMATED METHOD FOR ANALYZING AND INTERPRETATING AN ANTIMICROBIAL SUSCEPTIBILITY TEST

(30) Priorité: 06.10.2014 FR 1459559
(43) Date de publication de la demande: 16.08.2017
(73) Titulaire: Fondation Méditerranée Infection, 13005 Marseille (FR); Université d'Aix-Marseille, 13284 Marseille Cedex 07 (FR); Assistance Publique Hôpitaux de Marseille, 13005 Marseille (FR); Centre National de la Recherche Scientifique (C.N.R.S.), 75016 Paris (FR); INSERM (Institut National de la Santé et de la Recherche Médicale), 75013 Paris (FR)
(72) Inventeur: ROLAIN, Jean-Marc, F-13006 Marseille (FR); RAOULT, Didier, F-13008 Marseille (FR); LE PAGE, Stéphanie, F-13007 Marseille (FR); BUFFET, Sylvain, F-13011 Marseille (FR)
(74) Mandataire: Cabinet Beau de Loménie
(86) Numéro de dépôt international: PCT/FR2015/052667
(87) Numéro de publication internationale: WO 2016/055724

(56) Documents cités:
- SALGADO L ET AL: "Automatic antibiograms inhibition halo determination through texture and directional filtering analysis", PROCEEDINGS 2001 INTERNATIONAL CONFERENCE ON IMAGE PROCESSING. ICIP 2001 - THESSALONIKI, GREECE, OCT. 7 - 10, 2001; [INTERNATIONAL CONFERENCE ON IMAGE PROCESSING], INSTITUTE OF ELECTRICAL AND ELECTRONICS ENGINEERS, NEW YORK, NY, vol. 2, 7 October 2001 (2001-10-07), pages 629 - 632, XP010563842, ISBN: 978-0-7803-6725-8, DOI: 10.1109/ICIP.2001.958572
- E. KENT KORGENSKI ET AL: "Evaluation of the BIOMIC Video Reader System for Determining Interpretive Categories of Isolates on the Basis of Disk Diffusion Susceptibility Results", JOURNAL OF CLINICAL MICROBIOLOGY, 1 January 1998 (1998-01-01), UNITED STATES, pages 302 - 304, XP055196269, Retrieved from the Internet <URL:http://jcm.asm.org/content/36/1/302.abstract> [retrieved on 20150616]
- ANTONE A. MEDEIROS ET AL: "Evaluation of the Sirscan Automated Zone Reader in a Clinical Microbiology Laboratory", JOURNAL OF CLINICAL MICROBIOLOGY, 1 April 2000 (2000-04-01), UNITED STATES, pages 1688 - 1693, XP055196272, Retrieved from the Internet <URL:http://jcm.asm.org/content/38/4/1688.abstract> [retrieved on 20150616]
- M. KOLBERT ET AL: "Evaluation of the OSIRIS video reader as an automated measurement system for the agar disk diffusion technique", CLINICAL MICROBIOLOGY AND INFECTION, vol. 10, no. 5, 1 May 2004 (2004-05-01), pages 416 - 420, XP055196281, ISSN: 1198-743X, DOI: 10.1111/j.1469-0691.2004.00885.x
- F. BERT ET AL: "Evaluation and Updating of the Osiris Expert System for Identification of Escherichia coli -Lactam Resistance Phenotypes", JOURNAL OF CLINICAL MICROBIOLOGY, vol. 43, no. 4, 1 April 2005 (2005-04-01), pages 1846 - 1850, XP055196292, ISSN: 0095-1137, DOI: 10.1128/JCM.43.4.1846-1850.2005
- ON BEHALF OF THE STUDY GROUP 'ANTIMICROBIAL RESISTANCE IN INDONESIA: ET AL: "Comparison of the accuracy of disk diffusion zone diameters obtained by manual zone measurements to that by automated zone measurements to determine antimicrobial susceptibility", JOURNAL OF MICROBIOLOGICAL METHODS, ELSEVIER, AMSTERDAM, NL, vol. 75, no. 2, 1 October 2008 (2008-10-01), pages 177 - 181, XP024528578, ISSN: 0167-7012, [retrieved on 20080627], DOI: 10.1016/J.MIMET.2008.05.020
- STÉPHANIE LE PAGE ET AL: "Real-time video imaging as a new and rapid tool for antibiotic susceptibility testing by the disc diffusion method: A paradigm for evaluating resistance to imipenem and identifying extended-spectrum [beta]-lactamases", INTERNATIONAL JOURNAL OF ANTIMICROBIAL AGENTS, vol. 45, no. 1, 14 October 2014 (2014-10-14), pages 61 - 65, XP055195854, ISSN: 0924-8579, DOI: 10.1016/j.ijantimicag.2014.08.014

## Description

La présente invention concerne une méthode permettant l'automatisation de la lecture et de l'interprétation des tests de sensibilité des microorganismes aux antimicrobiens, dans le domaine de la microbiologie clinique.

Cette invention peut être appliquée aux tests de sensibilité des bactéries aux antibiotiques dénommés antibiogrammes en bactériologie principalement, mais également aux autres domaines de l'infectiologie tels que les tests de sensibilité des champignons aux antifongiques dénommés antifongigrammes pour la mycologie.

Dans la présente description, on utilise le terme générique « antibiogramme » indifféremment aussi bien pour les antibiogrammes de composés antibiotiques vis-à-vis des bactéries que pour les antifongigrammes de composés antifongiques vis à vis de champignons.

Un antibiogramme est une technique de laboratoire visant à tester la sensibilité d'une souche bactérienne vis-à-vis d'un ou plusieurs antibiotiques supposés ou connus en évaluant l'inhibition de la pousse de la bactérie. Le principe consiste à placer une culture de bactéries en présence du ou des antibiotiques et à observer les conséquences sur le développement et la survie de celle-ci. Pour ce faire, on peut par exemple placer plusieurs pastilles imbibées d'antibiotiques sur une souche bactérienne déposée sur un milieu de culture solide. Il se produit alors une diffusion de l'antibiotique à partir de la pastille ce qui crée un gradient de concentration autour de la pastille et une zone d'inhibition de croissance visible à l'œil nu sous forme d'auréole foncée autour de la pastille si la bactérie est sensible à l'antibiotique. Il existe trois types d'interprétation selon le diamètre du cercle de la zone d'inhibition qui entoure le disque de la pastille d'antibiotique : souche ou bactérie sensible, intermédiaire ou résistante.

Les pastilles et leur dose variable en antibiotique sont standardisées selon les recommandations des systèmes experts de l'EUCAST (The European Committee on Antimicrobial Susceptibility Testing : www.eucast.org) [Winstanley T, Courvalin P (2011) Expert systems in clinical microbiology. Clin Microbiol Rev 24: 515-556. 24/3/515 [pii];10.1128/CMR.00061-101, et Leclercq R, Canton R, Brown DF, Giske CG, Heisig P, MacGowan AP, Mouton JW, Nordmann P, Rodloff AC, Rossolini GM, Soussy CJ, Steinbakk M, Winstanley TG, Kahlmeter G (2013) EUCAST expert rules in antimicrobial susceptibility testing. Clin Microbiol Infect 19: 141-160. 10.1111/j.1469-0691.2011.03703.x]) et/ou de la CA-SFM (Comité de l'Antibiogramme de la Société Française de Microbiologie : www.sfm-microbiologie.org). Les pastilles d'antibiotiques sont en matériaux substrat renfermant une concentration connue d'antibiotique et apte à le diffuser tel que du papier buvard imprégné de concentrations d'antibiotiques variant de 1µg à 500µg environ en fonction des antibiotiques et sont commercialisés par les sociétés i2a (Montpellier, France), BD (Becton Dickinson, Etats-Unis), Bio-Rad (Hercules, Etats-Unis), Biomerieux (Marcy l'Etoile, France).

Les différents disques d'antibiotiques utilisés pour réaliser l'antibiogramme en phase solide sur gélose portent les dénominations des antibiotiques sous forme d'abréviations normalisées sous forme de code à 1 à 3 lettres inscrites sur la face supérieure de la pastille.

La lecture des résultats consiste essentiellement dans la mesure des diamètres des auréoles (zones d'inhibition de croissance de la souche microbienne). Plus le diamètre d'inhibition est grand plus la bactérie est sensible à l'antibiotique testé et vice versa. Les dosages des pastilles sont déterminés pour permettre d'obtenir des diamètres d'auréoles proportionnels aux concentrations inhibitrices minimales (CMI) de l'antibiotique concerné vis-à-vis de la bactérie testée.

Le diamètre d'inhibition est mesuré en mm soit de façon manuelle avec une règle ou avec un lecteur qui permet la lecture du diamètre en automatique. Ce lecteur automatique comprend un scanner équipé d'un appareil photo et d'un détecteur de pastilles d'antibiotiques et des zones d'inhibition se situant autour de ces disques.

Les antibiogrammes réalisés par diffusion sur milieu gélosé sont interprétés suivant les recommandations des systèmes experts de l'EUCAST et/ou de la CA-SFM.

Pour l'interprétation, il existe différents tableaux ou abaques en fonction des bactéries et antibiotiques où figurent des concentrations critiques inférieures et respectivement supérieures ainsi que les diamètres critiques supérieurs et respectivement inférieurs de la zone d'inhibition pour une concentration donnée du dit antibiotique dans la pastille. Chaque antibiotique présente vis-à-vis de chaque bactérie:
- une concentration critique inférieure (et un diamètre critique supérieur) en dessous de laquelle (et au-dessus duquel respectivement) la bactérie est considérée sensible pour l'antibiotique donné (indiqué par la lettre S), et
- une concentration critique supérieure (et un diamètre critique inférieur) au-dessus de laquelle (et au-dessous duquel respectivement) la bactérie est considérée résistante pour l'antibiotique donné (indiqué par la lettre R), et
- entre les deux concentrations, la bactérie sera dite intermédiaire (indiqué par la lettre I).

Ainsi, chaque bactérie vis-à-vis de chaque antibiotique, est caractérisée en S, I ou R vis à vis d'une bactérie donnée. Pour savoir si la bactérie est résistante à une famille d'antibiotique donnée, on considère le plus souvent qu'il suffit d'en tester un seul de cette famille pour connaître son profil. Une mesure d'un diamètre d'inhibition va donc permettre l'interprétation d'une classe thérapeutique d'antibiotiques. Cependant, une seule famille d'antibiotiques n'est pas suffisante pour connaître le phénotype complet de la bactérie en termes de résistance ou de sensibilité aux différents antibiotiques. C'est pourquoi, dans un antibiogramme on teste une pluralité d'antibiotiques en déposant une pluralité de pastilles contenant des antibiotiques de classes ou familles ou sous-familles différentes pour obtenir un phénotype de résistance ou de sensibilité aux différentes familles ou sous-familles d'antibiotiques.

L'antibiogramme permet au médecin de choisir le bon antibiotique, ou l'association d'antibiotiques permettant de traiter efficacement un patient.

Pour pouvoir connaître le phénotype complet d'une souche bactérienne donnée, il faut donc tester différents antibiotiques appartenant à des classes ou familles ou sous-familles thérapeutiques différentes et dont on connaît les mécanismes de résistances les plus fréquents. Les principaux antibiotiques (avec leurs codes entre parenthèses) relevant de chaque familles ou sous familles suivantes sont listés ci-après:
1) Famille : B-Lactamines :
   1.1) Sous familles des Pénicillines :
      1.1.1) Pénicillines du groupe G : Pénicilline G (P),
      1.1.2) Pénicillines M : Oxacilline (OX ou OXA),
      1.1.3) Pénicillines du groupe A ou aminopénicillines: Amoxicilline (AMX) et Ampicilline (AM) ;
      1.1.4) Pénicillines à spectre antibactérien très large :
         - inhibiteur de béta-lactamase: association Amoxicilline + acide clavulanique (AMC)
         - Carboxypénicillines: Ticarciline (TIC), l'association Ticarciline + acide clavulanique (TCC),
         - Uréidopénicillines : association Pipéracilline + Tazobactam (TZP),
   1.2) Sous familles des Céphalosporines :
      1.2.1) Céphalosporines de 1^{ère} génération: Céfalotine (CF),
      1.2.2) Céphalosporines de 2^{ième} génération: Céfoxitine (du type céphamycines)(FOX), Cefuroxime (CXM),
      1.2.3) Céphalosporine de 3^{ième} génération: Ceftriaxone (CRO), Céfotaxime (CTX), Ceftazidime (CAZ),
      1.2.4) Céphalosporines de 4^{ième} génération à spectre élargi: Céfépime (FEP), Cefpirome (CPO),
   1.3) Sous-famille des Monobactames: Aztréonam (ATM),
   1.4) Sous familles des Carbapénèmes: Imipenème (IMP ou IPM) et Ertapénème (ERT),
2) Famille des quinolones:
   2.1) Quinolones de 1ère génération: Acide nalidixique (NA),
   2.2) Quinolones de 2ième génération du type fluoroquinolones: Ciprofloxacine (CIP), Ofloxacine (OFX), Péfloxacine (PEF), Lévofloxacine (LVX), Moxifloxacine (MXF), Norfloxacine (NOR),
3) Famille des Aminosides: Gentamicine (GEN ou GM), Amikacine (AK ou AN), Tobramycine (TOB ou TM), Kanamycine (K),
4) Famille des Rifamycines: Rifampicine (RA),
5) Famille des Glycopeptides: Vancomycine (VA ou VAN) et Téicoplanine (TEC),
6) Famille des Sulfamides et diaminopyrimidines: l'association Triméthoprime + sulfamethoxazole = Cotrimoxazole (SXT),
7) Famille des Macrolides: Erythromycine (E), Télithromycine (TEL),
8) Famille des lincosanides : lincomycine (LIN) et clindamycine (CLI ou DA),
9) Famille des Synergistines ou Streptogramines : Pristinamycine (PT),
10) Famille des Tétracyclines: Doxycycline (DO ou TE),
11) Famille des polymyxines ou polypeptides cycliques : Colistine (COL ou CS),
12) Famille des furanes : nitrofurane-furane (FT)- Nitrofurantoïne (FUR ou NT),
13) Famille des Phénicolés : Chloramphénicol (C),
14) Famille des Fusidanines : Acide Fluidique (FA),
15) Famille des antibiotiques phosphoniques : Fosfomycine (FF ou FOS),
16) Famille des Oxazolidinones : Linézolide (LNZ),
17) Famille des Lipopeptides cycliques : Tigécycline (TGC).

L'interprétation des résultats utilisant des tableaux ou des abaques de données indiquant pour les différentes bactéries les diamètres critiques correspondants aux concentrations critiques pour des pastilles contenant des concentrations déterminées d'antibiotiques ou une association d'antibiotiques déterminés, provenant d'organismes officiels comme le CA-SFM (Comité de l'Antibiogramme de la Société Française de Microbiologie) ou encore de l'EUCAST (European Comitee on Antimicrobial Susceptibility testing) telle que décrite ci-dessus est une procédure longue et complexe qui représente une contrainte importante.

Dans l'état de l'art (1-6), on connait différentes techniques automatisées d'analyses d'antibiogrammes impliquant des acquisitions d'images de la zone des disques de diffusion autour des antibiotiques dans un substrat tel qu'une pastille à l'aide de logiciels qui réalisent le calcul de diamètre desdites zones de diffusion et le comparent au diamètre de référence obtenu par les méthodes standards utilisant des calibres (méthode dite « NCCLS »). On peut notamment faire référence au document Salgado et al, « Automatic antibiograms inhibition halo détermination through texture and directional filtering analysis », 2001, Institute of electrical and electronics engineers, page 629-632 qui décrit une méthode d'analyse et d'interprétation automatisée d'un antibiogramme d'un échantillon bactérien impliquant l'acquisition d'images de la zone des disques de diffusion autour des antibiotiques d'une pastille à l'aide d'un logiciel réalisant le calcul du diamètre des auréoles de diffusion.

Le but de la présente invention est de fournir une méthode plus simple à réaliser et plus fiable permettant de s'affranchir de la lecture et interprétation des diamètres des auréoles des zones d'inhibition autour des pastilles une à une selon les critères élaborés par le CA-SFM (Comité de l'Antibiogramme de la Société Française de Microbiologie) ou encore de l'EUCAST (European Comitee on Antimicrobial Susceptibility testing).

On a découvert selon la présente invention que les images d'antibiogrammes différents pouvait être caractéristiques d'un phénotype et que l'on peut reconnaître un phénotype de résistance de bactérie vis à vis des antibiotiques par comparaison de photos à l'aide d'une banque d'images photographiques de l'antibiogramme de référence sans nécessiter de les interpréter selon les données d'interprétation de l'EUCAST ou CA-SFM ; et ceci, bien qu'il existe des différences de diamètres ou de formes entre 2 images globales d'antibiogrammes de différentes photos de même phénotype ; sous réserve que pour un phénotype donné, on dispose d'une collection de photos de plusieurs bactéries de la même espèce et du même phénotype.

Plus précisément, pour ce faire la présente invention fournit une méthode d'analyse et d'interprétation automatisée d'un antibiogramme d'un échantillon de microorganisme à analyser, de préférence une bactérie, permettant de déterminer un phénotype de résistance ou de sensibilité à au moins un composé antimicrobien, de préférence une pluralité de composés antimicrobiens, de préférence des composés antibiotiques, le dit phénotype étant choisi parmi une pluralité de phénotypes de référence différents pour chacune des espèces de microorganismes de référence d'une pluralité d'espèces de microorganismes de référence différentes dans lequel:
a) l'antibiogramme de l'échantillon du microorganisme à analyser, de préférence appartenant à l'un des dites espèces de microorganismes de référence, est réalisé selon une méthodologie déterminée sur un milieu de culture solide, méthodologie comprenant:
   - une concentration de microorganisme, de préférence de bactérie (UFC/mL), déterminée de l'échantillon de microorganisme, de préférence bactérie, déposée sur le dit milieu de culture solide,
   - un nombre n déterminé d'une pluralité de substrats, de préférence n= 5 à 20, de préférence encore n= 6 à 16, une disposition déterminée et une forme déterminée de substrats, de préférence en forme de pastilles, contenant chacun(e) une concentration déterminée de composé(s) antimicrobien(s) déterminé(s) et aptes à diffuser un ou des composés antimicrobien(s) déterminés différents pour chacun des n dits substrats déposés sur le dit milieu de culture solide, et
   - des conditions et durée d'incubation déterminées de l'échantillon de microorganisme déposé sur le dit milieu de culture solide, de préférence de 18 à 24h à une température de 35- à 37°C, et
b) on réalise l'acquisition d'une image photographique de l'antibiogramme obtenu à l'étape a), de préférence à l'aide d'un appareil de capture d'image photographique comprenant un scanner et appareil photographique, et
c) on détermine le dit phénotype de l'échantillon de microorganisme à analyser, de préférence bactérie, sans mesurer directement les diamètres d'inhibition sur l'antibiogramme, en comparant avec un logiciel de reconnaissance d'image, l'image photographique obtenue à l'étape b) avec des images de référence d'antibiogrammes de référence, d'une base de données constituée des dites images de référence, telles que la dite base de donnée comprend une pluralité d'au moins p images de référence différentes d'antibiogrammes de p souches différentes du même microorganisme pour chaque phénotype de référence de chaque espèce de microorganisme de référence, de préférence p étant au moins égal à 5, de préférence encore p étant au moins égal à 10.

De préférence tous les antibiogrammes de référence sont réalisés selon la même dite méthodologie que l'antibiogramme à analyser.

De préférence, les dites d'images de référence et l'image à analyser sont réalisées avec le même appareil de capture d'image photographique et dans les mêmes conditions, notamment même distance, même luminosité, même fond, de préférence un fond plat et noir, et même résolution.

On comprend que :
- les antibiogrammes de référence sont réalisés selon la même dite méthodologie que l'antibiogramme à analyser en termes de nombre, disposition et forme des pastilles ainsi que concentration bactérienne et conditions d'incubation, excepté qu'ils ont des pastilles contenant des composés antibiotiques et/ou des concentrations de composés antibiotiques en partie au moins différents pour les différents phénotypes de référence, et
- les phénotypes de référence des différentes souches de références de chaque espèce de bactérie de référence de la dite base de donnée sont déterminés manuellement par les méthodes usuelles standard par mesure du diamètre de l'auréole de zone d'inhibition de croissance autour des pastilles d'antibiotiques et à l'aide d'abaques indiquant les valeurs de diamètres critiques correspondants aux concentrations critiques inférieures et concentrations critiques supérieures pour une concentration donnée d'antibiotique(s) dans une dite pastille permettant de classer la bactérie de l'échantillon analysée comme étant :
- sensible à l'antibiotique ou combinaison d'antibiotiques contenu dans la dite pastille, si le diamètre de l'auréole d'inhibition entourant la pastille est supérieure au diamètre correspondant à la concentration critique inférieure,
- résistante à l'antibiotique ou combinaison d'antibiotiques contenu dans la dite pastille, si le diamètre de l'auréole d'inhibition entourant la pastille est inférieur au diamètre correspondant à la concentration critique supérieure, et
- intermédiaire par rapport à l'antibiotique ou combinaison d'antibiotiques contenu dans la dite pastille, si le diamètre de l'auréole d'inhibition entourant la pastille est compris entre les valeurs de diamètres critiques correspondants aux concentrations critiques inférieures et concentrations critiques supérieures ;
- la comparaison de l'image photographique obtenue à l'étape b) avec les dites images de référence permet de déterminer le phénotype de l'échantillon de bactérie à analyser comme étant un dit phénotype de référence d'une espèce de bactérie de référence si l'image photographique obtenue à l'étape b) est identique ou au moins suffisamment similaire à celles d'une dite image de référence correspondant au dit phénotype de référence d'une espèce de bactérie de référence. Un taux minimal de similitude peut être déterminé expérimentalement à l'aide de la pluralité d'images de référence correspondant à un même phénotype d'une même espèce de bactérie de références.

La méthode selon l'invention procède instantanément par reconnaissance globale d'image sans calcul des différents diamètres d'auréoles comme fait manuellement dans la méthode classique d'interprétation des antibiogrammes. Les inventeurs ont découvert qu'en dépit du fait qu'un diamètre d'auréole d'inhibition peut varier dans une fourchette de valeurs tout en relevant du même phénotype par rapport à l'antibiotique concerné, en pratique l'analyse de multiples zones ou auréoles combinée à la multiplicités des images de références relevant du même phénotype permet une interprétation correcte de l'antibiogramme.

Un autre avantage de la méthode selon la présente invention est qu'elle permet de détecter automatiquement et prendre en compte des phénotypes dit de synergie entre deux antibiotiques contenus dans deux pastilles différentes disposées cote à cote normalement résistants vis à vis de cette souche. Les synergies sont générées avec la méthode sur gélose par diffusion des antibiotiques sur cette gélose du fait que si la distance entre les 2 antibiotiques est suffisamment proche (entre 2-2,5 cm pour les bactéries Gram négatives) ils vont pouvoir diffuser sur une partie commune de la gélose et c'est donc l'association des 2 antibiotiques à cet endroit précis qui permet le cas échéant de visualiser l'apparition d'une nouvelle zone d'inhibition qui est la zone de synergie.

Dans ce cas de synergie, en effet, il est possible de visualiser des mécanismes de synergies entre deux antibiotiques car, une zone d'inhibition supplémentaire, généralement entre les deux antibiotiques en question, apparaît. Il peut s'agir d'un élargissement du diamètre autour du disque A vers le disque B si A et B présentent une synergie entre eux. Donnant une zone d'inhibition sous forme sensiblement ovale dénommée « bouchon de champagne » entre les deux auréoles autour des pastilles A et B. De nouvelles synergies pourraient être découvertes à l'avenir ; la non similitude d'une image de synergie si elle est nouvelle pourrait être détectée grâce à la méthode selon l'invention.

De préférence, à l'étape c), on compare avec le logiciel de reconnaissance d'image, la forme du contour des auréoles correspondants aux zones d'inhibition autour des substrats, de préférence des pastilles, d'antibiotiques et de préférence aussi les lettres des codes et chiffres de dosage des antibiotiques indiqués sur les pastilles d'antibiotiques, et de préférence encore le logiciel reconnaît l'ordre dans lequel sont disposés les pastilles.

Des logiciels de ce type sont mis en oeuvre pour reconnaître une espèce de plante par l'analyse de l'image du contour de sa feuille. On connaît aussi un logiciel de reconnaissance d'images dénommé XnView librement accessible décrit ci-après qui est aussi particulièrement adapté ici car il rend ainsi compte de la variation des caractéristiques déterminantes à savoir les différents diamètres des différentes auréoles d'une part et d'autre part de leur chevauchement éventuel sous forme de zone de synergie.

Plus particulièrement, on détermine un pourcentage de similitude minimal admissible entre une image à analyser et une image de référence, dénommé ci-après seuil de similitude, de sorte que la bactérie de l'échantillon à analyser sera déterminée comme présentant un dit phénotype de référence d'une dite bactérie de référence si le logiciel de reconnaissance évalue un pourcentage de similitude entre l'image de l'antibiogramme de la bactérie de l'échantillon à analyser et une image de référence correspondant audit phénotype de référence de la dite espèce de bactérie de référence, au moins égal à un pourcentage de similitude seuil admissible minimal de préférence un pourcentage de similitude seuil admissible minimal d'au moins 70% de préférence au moins 80%.

Le logiciel de reconnaissance permet de déterminer un dit pourcentage de similitude minimal admissible entre une image à analyser et une image de référence, dénommé ci-après seuil de similitude.

Dans un mode de réalisation, on peut donc déterminer un seuil de similitude commun applicable pour tous les phénotypes de référence.

Dans un autre mode de réalisation, on détermine un pourcentage de similitude minimal admissible entre les dites images de référence différentes des différents antibiogrammes des p souches différentes, pour chaque phénotype de référence de chaque espèce de bactérie de référence, ci-après dénommé seuil de similitude de référence, de préférence on détermine un pourcentage de seuil de similitude de référence, correspondant au pourcentage de similitude le plus bas entre les deux images de référence les plus distinctes pour chaque phénotype de référence de chaque espèce de bactérie de référence, de sorte que la bactérie de l'échantillon à analyser sera déterminée comme présentant un dit phénotype de référence d'une dite bactérie de référence si le logiciel de reconnaissance évalue un pourcentage de similitude au moins égal audit seuil de similitude de référence entre l'image de l'antibiogramme de la bactérie de l'échantillon à analyser et une image de référence correspondant audit phénotype de référence de la dite espèce de bactérie de référence.

Dans une variante de réalisation, le dit phénotype de référence est une classification de résistance ou sensibilité pour un antibiotique ou une association d'antibiotiques donnée.

Dans cette variante, l'antibiogramme peut être un E-test, le logiciel peut lire les valeurs de CMI mentionnées sur des bandelettes contenant un gradient de concentrations d'un antibiotique seul, la CMI étant la valeur au niveau de l'extrémité plus étroite de l'auréole ovale entourant la bandelette. Le procédé selon l'invention permet d'éviter les erreurs fréquentes de lecture de CMI à l'oeil nu des opérateurs.

Dans une autre variante de réalisation, le phénotype de référence est une classification de résistance ou sensibilité pour une pluralité d'antibiotiques et/ou d'association d'antibiotiques, de préférence dans des substrats en forme de pastilles.

De préférence, un dit phénotype de référence comprend un phénotype de synergie entre deux antibiotiques correspondant à deux antibiotiques pour lesquels la bactérie de référence est résistante lorsqu'il sont pris séparément et pour lesquels la bactérie de référence est sensible lorsqu'ils sont pris en association. Ce phénotype de synergie se reconnait par l'apparition d'une zone d'inhibition dans l'espace entre deux pastilles correspondant à deux antibiotiques pour lesquels la bactérie de référence est résistante.

Plus particulièrement, les dites espèces de bactéries de référence sont choisies parmi les bactéries les plus fréquentes, à savoir représentant environ 80% des bactéries responsables de pathologies humaines les plus fréquentes, comprenant au moins *Escherichia coli, Klebsiella pneumoniae, Proteus mirabilis, Klebsiella oxytoca, Enterobacter cloacae, Enterobacter aerogenes, Enterococcus faecalis, Pseudomonas aeruginosa, Staphylococcus aureus et Staphylococcus epidermidis.*

Plus particulièrement encore, les antibiogrammes sont réalisés à partir de pastilles comprenant les antibiotiques suivants et avec les marquages suivants correspondants aux antibiotiques suivants: AMX pour amoxicilline; AMC pour Amoxicilline-acide clavulanique; GEN pour gentamicine; SXT pour Triméthoprime-sulfamethoxazole (Cotrimoxazole); TIC, pour Ticarcilline;TCC pour Ticarcilline-acide clavulanique; TZP pour Piperacilline-tazobactam; CAZ pour Ceftazidime; IMP pour Imipenème; COL ou CL pour Colistine; FOX pour Céfoxitine; VAN ou VA pour Vancomycine; TEC pour Téicoplanine; CLI ou DA pour Clindamycine; CRO pour Ceftriaxone; FEP pour Céfépime; L ou LIN pour Lincomycine; FUR ou NT pour Nitrofurantoïne, CIP pour Ciprofloxacine; OFX pour l'Ofloxacine; CTX pour Céfotaxime; AK ou AN pour Amikacine; TOB ou TM pour Tobramycine; FOX pour Céfoxitine; ATM pour Aztréonam; ERT pour Ertapénème; FF ou FOS pour Fosfomycine; DO pour Doxycycline; K pour Kanamycine; L ou LIN pour Lincomycine; LNZ pour Linézolide; NA pour Acide Nalidixique; NOR pour Norfloxacine; MET pour Métronidazole; MEM pour Méropénème; PT pour Pristinamycine; P pour Pénicilline G; RA pour Rifampicine; TGC pour Tigécycline; TEL pour Télithromycine; E pour Erythromycine, OX ou OXA pour Oxacilline.

Plus particulièrement encore, les inventeurs ont déterminé des phénotypes de référence particuliers plus particulièrement appropriés pour tester des bactéries responsables de pathologies humaines détectables notamment dans les urines et traités par les antibiotiques ci-dessus, le phénotype de référence étant une classification de résistance ou choisie parmi au moins les phénotypes suivants:
- Phénotype dénommé «sauvage»: bactérie qui ne possède aucune résistance acquise aux antibiotiques, la dite bactérie pouvant posséder des résistances naturelles,
- Phénotype dénommé «Pénicillinase de bas niveau»: bactérie productrice d'une pénicillinase capable d'inhiber la croissance bactérienne par de faibles concentrations en antibiotique de la famille des pénicillines, à savoir des concentrations inférieure à 8 mg/L.
- Phénotype dénommé «Pénicillinase résistante aux inhibiteurs»: bactérie produisant une pénicillinase résistant à tous les inhibiteurs de pénicillinase sensés capable d'inactiver cette enzyme;
- Phénotype dénommé «Pénicillinase de haut niveau»: bactérie productrice d'une pénicillinase capable d'inhiber la croissance bactérienne par de fortes concentrations en antibiotique de la famille des pénicillines, à savoir des concentrations supérieures à 8 mg/L.
- Phénotype dénommé «Béta-Lactamase à Spectre étendu» (BLSE): bactérie productrice de Béta-lactamase;
- Phénotype dénommé «Céphalosporinase haut niveau»: bactérie produisant une céphalosporinase capable d'inhiber la croissance bactérienne par de fortes concentrations en antibiotique de la famille des céphalosporines, à savoir des concentrations supérieures à 2 mg/L.
- Phénotype dénommé «Carbapénèmase»: bactérie produisant une carbapénèmase ;
- Phénotype dénommé «Perméabilité sélective à l'imipenème»: bactérie possède une sensibilité intermédiaire vis à vis de l'imipenème;
- «Résistance à la méthicilline» (ancien nom d'une pénicilline du groupe M, actuellement remplacée par l'Oxacilline qui est une pénicilline appartenant à la sous-famille des pénicillines du groupe M) bactérie résistant à la méthicilline (= résistante à l'Oxacilline) par acquisition d'une nouvelle cible dépourvue d'affinité pour les B-Lactamines (PLP2a);
- Phénotype dénommé «résistance aux fluoroquinolones»: bactérie dont la croissance n'est pas inhibée en présence de composés antibiotique de la famille des fluoroquinolones,
- Phénotype dénommé « résistance aux aminosides»: bactérie dont la croissance n'est pas inhibée en présence de composés antibiotique de la famille des aminosides,
- Phénotype dénommé «résistance aux macrolides»: bactérie dont la croissance n'est pas inhibée en présence de composés antibiotique de la famille des macrolides,
- Phénotype dénommé «résistance au Cotrimoxazole»: bactérie dont la croissance n'est pas inhibée en présence de Cotrimoxazole,
- phénotype dénommé «résistance à la Rifampicine»: bactérie dont la croissance n'est pas inhibée en présence de Rifampicine,
- Phénotype dénommé «Phénotype atypique»: phénotype qui n'est pas connu ou pas déterminable.

Une pénicillinase est une enzyme capable de détruire (hydrolyser) la pénicilline et de la rendre inactive vis à vis de la bactérie.

Une céphalosporinase est une enzyme capable de détruire (hydrolyser) les céphalosporines et de les rendre inactives vis à vis de la bactérie.

Une carbapénèmase est une enzyme capable de détruire (hydrolyser) les carbapénèmes et de les rendre inactives vis à vis de la bactérie.

Une Béta-lactamase est une enzyme capable d'hydrolyser les pénicillines, les céphalosporines et les monobactames telles que l'aztréonam, mais pas les carbapénèmes ni les céphamycines telles que Céfoxitine.

Plus particulièrement encore, l'antibiogramme analysé et/ou la dite base de donnée comprend les phénotypes de références pour les espèces de bactéries de référence correspondant à des résistances (I,R) ou sensibilités (S) suivantes vis-à-vis des concentrations des 6 composés antibiotiques choisis parmi les groupes a1) à a6) suivants, de préférence disposés en cercle dans l'ordre suivant pour chaque groupe:
a1) AMX, AMC, TCC, CRO, FEP et IMP,
a2) AMX, AMC, TZP, CRO, FEP et IMP,
a3) AMC, TZP, CRO, FEP, IMP et COL,
a4) AMX, GEN, LIN,FUR, VAN et TEC,
a5) TIC, TCC, TZP, FEP, CAZ et IMP,
a6) FOX, CLI, SXT, GEN, VAN et TEC.

Ces groupes a1) à a6) sont plus particulièrement pertinents au regard des phénotypes suivants pour les bactéries listées ci-dessus :
- Phénotype «sauvage»,
- Phénotype «Pénicillinase de bas niveau»,
- Phénotype «Pénicillinase résistante aux inhibiteurs»,
- Phénotype «Pénicillinase de haut niveau»,
- Phénotype «Béta-Lactamase à Spectre étendu» (BLSE),
- Phénotype «Céphalosporinase haut niveau»,
- Phénotype «Carbapénèmase»,
- Phénotype «Perméabilité sélective à l'imipenème»,
- «Résistance à la méthicilline», et
- Phénotype «Phénotype atypique».

Plus particulièrement encore, l'antibiogramme analysé et/ou la dite base de donnée comprend les phénotypes de références suivants pour les espèces de bactéries de référence suivantes correspondant aux résistances (I/R)/ ou sensibilités (S) suivantes vis-à-vis des 16 composés antibiotiques choisis parmi les groupes b1) et b2) suivants, de préférence dont les pastilles sont disposées en quadrillage dans l'ordre suivant à partir de la première rangée à droite avec 4 rangées de 4 composés alignées en colonnes pour chaque groupe :
b1) CIP, OFX, TIC, COL, IMP, CTX, TCC, AK, SXT, AMC, CRO, TOB, AMX, ATM, FOX et GEN,
b2) SXT, DA, FOX, OXA, PT, GEN, CIP, RA, TEC, VAN, LNZ, FF, TOB, E, DO et NT.

Ces groupes b1 et b2 sont plus particulièrement pertinents au regard des phénotypes suivants pour les bactéries listées ci-dessus:
- Phénotype « sauvage»,
- Phénotype associant «Pénicillinase de haut niveau» + «résistance aux fluoroquinolones» + «résistance au Cotrimoxazole» + «résistance aux aminosides», et
- Phénotype associant «Céphalosporinase » + «résistance aux fluoroquinolones» + «résistance au Cotrimoxazole» + «résistance aux aminosides», et
- Phénotype associant «Céphalosporinase » + «résistance au Cotrimoxazole»+ « résistance aux aminosides», et
- Phénotype associant «Résistance à la méthicilline» + «résistance aux fluoroquinolones» + «résistance à la rifampicine» + «résistance aux macrolides» + «résistance aux aminosides».

Ces phénotypes de références à 16 pastilles ci-dessus sont plus, particulièrement utiles pour les infections dites profondes comme par exemple les septicémies.

Plus particulièrement encore, à l'étape a), on réalise les antibiogrammes de référence et de l'échantillon de bactérie à analyser, selon une méthodologie déterminée sur un milieu de culture solide de type gélose de Muller-Hinton, comprenant:
- un nombre (n), de 6 à 16 pastilles en forme de disques, à des concentrations en antibiotique de 1 à 300ug, disposées en cercle ou en quadrillage,
- une concentration bactérienne (UFC/mL) déterminée de l'échantillon à analyser ou de référence déposée sur le dit milieu de culture solide, correspondant à une turbidité de la suspension déposée correspondant à l'étalon de 0.5 dans la gamme de McFarland, et
- des conditions et durée d'incubation déterminée de préférence de 18 à 24h à 37°C.

Plus particulièrement encore, les pastilles sont disposées en cercle au nombre de 6 sur un milieu de culture ou disposées régulièrement en quadrillage dans des boites de forme carrée, de préférence au nombre de 16.

D'autres caractéristiques et avantages de la présente invention ressortiront mieux à la lecture de la description qui va suivre, faite de manière illustrative et non limitative, en référence aux dessins annexés sur lesquels :
- les figures 1A et 1B montre le principe de reconnaissance d'un contour d'image globale d'un antibiogramme; la figure 1A montrant le contour global de l'ensemble des zones d'inhibition 1 à 6 de l'image de la figure 1B ;
- les figures 2A à 2C représentent des images photographiques des antibiogrammes de souches *E. coli* de phénotype BLSE reconnues comme appartenant au même phénotype avec un taux de similitude de 87 à 100% par le procédé selon l'invention,
- les figures 2D et 2E représentent des images photographiques d'antibiogrammes de souches *E. coli* de phénotype Céphalosporinase reconnues comme appartenant au même phénotype avec un taux de similitude de 75% par le procédé selon l'invention,
- les figures 3A et 3B montrent deux photos d'antibiogrammes de 2 souches *d'Escherichia coli* productrices de pénicillinases de haut niveau et reconnues comme telle avec un taux de similitude de 80% par le procédé selon l'invention,
- les figures 4A et 4B montrent des photos d'antibiogrammes de phénotypes non reconnus comme appartenant à un même phénotype par le procédé selon l'invention, la figure 4A montrant une souche *E. Coli* sensible à tous les antibiotiques et la figure 4B, une souche *E. coli* résistante à tous les antibiotiques.
- la figure 5 représente une image de zone de synergie en forme de bouchon de champagne (A) pour une souche *E. coli* de phénotype BLSE entre les deux pastilles AMC et CRO ;
- les figures 6A et 6B montrent des photos d'antibiogrammes disposés en carré de 2 souches de *K. pneumoniae* de même phénotype Céphalosporinase reconnus comme telle avec un taux de similitude de 88%par le procédé selon l'invention.

Les recommandations actuelles des comités d'expert sont des livrets d'environ 100 pages qui comprennent l'interprétation SIR de dizaines d'antibiotiques pour les espèces bactériennes les plus communes. Pour une classe d'antibiotique donnée le biologiste peut décider de tester un ou plusieurs antibiotiques proposés dans ces recommandations. En pratique seuls quelques antibiotiques sont des antibiotiques clés pour l'interprétation et surtout sont utiles pour le clinicien qui par exemple s'il veut utiliser une pénicilline pour traiter son patient n'utilisera qu'une seule molécule dans cette liste. Dans l'exemple illustratif ci-après, les inventeurs ont déterminé 6 antibiotiques représentatifs des autres, pour déterminer certains phénotypes des 10 bactéries les plus répandues en pathologie humaine.

### 1) Réalisation de l'antibiogramme.

Les antibiogrammes ont été réalisés selon la méthodologie standard suivante.

### 1.1) préparation d'échantillon bactérien

La préparation de l'inoculum consiste à réaliser une suspension bactérienne directement à partir de colonies mises en solution dans des tubes d'eau stérile de sorte que la solution présente une turbidimétrie déterminée avec un spectrophotomètre, précise équivalente à celle de l'étalon 0,5 de la gamme de McFarland. Cette concentration de suspension bactérienne est standardisée car un inoculum plus concentré engendre des diamètres de zone d'inhibition plus petits et inversement.

Cette inoculum correspond à des concentrations d'environ 108 UFC/mL pour *Escherichia coli, Klebsiella pneumoniae, Proteus mirabilis, Klebsiella oxytoca, Enterobacter cloacae, Enterobacter aerogenes, Enterococcus faecalis, Pseudomonas aeruginosa, Staphylococcus aureus, et Staphylococcus epidermidis.* Il s'agit des 10 espèces bactériennes les plus fréquemment rencontrées dans les pathologies humaines.

1.2). L'échantillon bactérien est déposé sur un milieu de culture solide dans une boite de pétri de 90 mm de diamètre ou boite carrée de 120 mm de cote consistant en une gélose Mueller-Hinton spécifiques des antibiogrammes (EUCAST). Il doit idéalement être employé dans les 15 minutes qui suivent la préparation de la suspension.

L'ensemencement est réalisé par la méthode de diffusion sur milieu gélosé par inondation dans laquelle la suspension bactérienne est rajoutée en excès sur la gélose Mueller-Hinton. Après homogénéisation, l'excès de liquide est rejeté pour éviter une sur-inoculation des boites. Il est nécessaire d'attendre au préalable que la surface des géloses soit sèche pour éliminer toute trace d'humidité favorisant l'envahissement bactérien.

Le dépôt des disques de pastilles d'antibiotiques doit être réalisé dans un délai de 15 min suivant l'inoculation de la gélose. Le dépôt des disques imprégnés d'antibiotique se fait à l'aide d'un distributeur automatique qui dépose fermement les disques à la surface de la gélose inoculée et séchée. Le nombre de disques déposés par boite est limité du fait du chevauchement des zones d'inhibition et pour limiter les interférences entre les antibiotiques. Il est important que les diamètres d'inhibition soient mesurables. Les antibiogrammes sont ensuite incubés à 37°C pendant 18-24 heures.

Les pastilles d'antibiotiques sont en papier absorbant de haute qualité et mesurent 6 mm de diamètre imprégnés de concentrations d'antibiotiques sont de 1µg à 300µg environ en fonction des antibiotiques.

Dans les exemples ci-après, on met en oeuvre des disques de références commercialisés par les sociétés i2a (Montpellier, France), BD (Becton Dickinson, Etats-Unis), Bio-Rad (Hercules, Etats-Unis), Biomerieux (Marcy l'Etoile, France) contenant les antibiotiques suivants et portant les codes suivants et doses suivantes : AMX (Amoxicilline) de 25µg; AMC (Amoxicilline-acide clavulanique) de 20-10ug; GM pour gentamicine de 15 µg; SXT (Triméthoprime-sulfamethoxazole) de 1.25/23.75µg; TIC (Ticarcilline) de 75µg;TCC(Ticarcilline-acide clavulanique) de 75/10µg; TZP ( Piperacilline-tazobactam) de 75/10pg ; CAZ (Ceftazidime) de 30µg IMP ou IPM (Imipenème) de 10µg; COL (Colistine) de 50µg; FOX (Cefoxitine) de 30µg; VAN (Vancomycine) de 30µg; TEC (Teicoplanine) de 30µg; CLI ou DA (Clindamycine) de 2µg; CRO (Ceftriaxone) de 30µg; CIP (Ciprofloxacine) de 5µg ; OFX (Ofloxacine) de 5µg ; CTX (Céfotaxime) de 5µg ; AN ou AK (Amikacine) de 30µg ; TM (Tobramycine) de 10µg ; FOX (Céfoxitine) de 30µg ; ATM (Aztréonam) de 30µg ; ERT (Ertapénème) de 10µg ; FF (Fosfomycine) de 200µg ; DO (Doxycycline) de 30µg ; K (Kanamycine) de 1000µg ; L (Lincomycine) de 15µg ; LNZ (Linézolide) de 10µg ; AN (Acide Nalidixique) de 30µg; NOR (Norfloxacine) de 10µg ; PT (Pristinamycine) de 15µg; RA (Rifampicine) de 5µg ; et E (Erythromycine) de 15µg.

Plus précisément on prépare la gélose comme suit:
- on ensemence la gélose par 1 mL de suspension et on étale le volume avec un râteau du centre vers les bords jusqu'à ensemencement de la totalité de la surface;
- on laisse sécher de 3 à 5 minutes;
- on positionne les disques d'antibiotiques sur le fond de la boîte (à du bord minimum); en divisant la boîte autant de fois (maximum 6 en cercle pour une boîte de 90 mm, ou 16 pour une boîte de 120 mm) que de pastilles, soit régulièrement espacées disposés en cercle ou en quadrillage espacées de 2.5cm pour les boites rondes et de 3 cm pour les boites carrées.

- on laisse incuber 18 à 24 h à 37°C.

### 2) Lecture et interprétation des antibiogrammes :

Après le temps d'incubation, on voit apparaître des auréoles autour des disques d'antibiotiques. Ces zones correspondent aux diamètres d'inhibition de la croissance bactérienne. En effet pour chaque antibiotique présent sur la gélose, du fait du gradient de concentration en antibiotique, il y a une zone où la pousse est inhibée comme explicité précédemment. Les auréoles d'inhibition apparaissent plus foncées car les photos sont réalisées sur fond noir et donc le contraste est plus important dans les zones où il n'y a pas eu de croissance bactérienne.

Pour constituer une banque d'antibiogrammes de référence pouvant servir à constituer une base de données d'images de référence de phénotypes de références pour les bactéries de référence on a mesuré manuellement des diamètres des auréoles et utilisé les tableaux et abaques pour leur interprétation tel que le tableau A ci-après.

2.1) Si on prend l'exemple *d'Escherichia coli,* on cherche par exemple à connaître sa sensibilité vis à vis de différents carbapénèmes dont l'imipenème on trouve dans le tableau ci-dessous provenant des recommandations de l'EUCAST (The European Committee on Antimicrobial Susceptibility Testing : www.eucast.org ) [1] et de la CA-SFM (Comité de l'Antibiogramme de la Société Française de Microbiologie : www.sfm-microbiologie.org ):

**Tableau A**

| Carbapénèmes | Concentrations critiques (mg/L) | | Charge du disque (µg) | Diamètres critiques (mm) | |
|---|---|---|---|---|---|
| | S≤ | R> | | S≥ | R< |
| Doripénème | 1 | 2 | 10 | 24 | 21 |
| Ertapénème | 0,5 | 1 | 10 | 25 | 22 |
| Imipénème | 2 | 8 | 10 | 22 | 16 |
| Méropénème | 2 | 8 | 10 | 22 | 16 |

Pour une dose de 10 µg d'antibiotique, les diamètres critiques en mm sont compris entre < 16 et ≥ 22 mm ce qui signifie que si le diamètre est supérieur ou égal à 22 mm la souche est sensible à l'imipenème, si le diamètre est compris entre 16 et strictement inférieur à 22 mm la souche possède une sensibilité intermédiaire à l'antibiotique donné et si le diamètre est strictement inférieur à 16 mm la souche est résistante à l'imipenème.

2.2) Ainsi, avec 206 échantillons bactériens des bactéries de référence et pastilles d'antibiotiques listés au tableau B (avec 6 pastilles de 6 antibiotiques différents) ci-après, on a élaboré une banque d'échantillons de bactéries de référence avec les phénotypes de références suivants listés au tableau C1 ci-après.

**Tableau B**

| Bactérie (nombre de souches) | Panel des 6 antibiotiques de concentrations fixes | | | | | |
|---|---|---|---|---|---|---|
| *E. coli (42)* | AMX 25 µg | AMC 20/10 µg | TCC 75/10 µg | CRO 30 µg | FEP 30 µg | IMP 10 µg |
| *P.mirabilis (16)* | | | | | | |
| *K. pneumonia (38)* | AMC 20/10 µg | TZP 75/10 µg | CRO 30 µg | FEP 30 µg | IMP 10 µg | COL 50 µg |
| *K. oxytoca (6)* | | | | | | |
| *E.cloacae (8)* | | | | | | |
| *E.aerogenes (11)* | | | | | | |
| *P.aeruginosa* (.35) | TIC 75 µg | TCC 75/10 µg | TZP 75/10 µg | FEP 30 µg | CAZ 30 µg | IMP 10 µg |
| *E.faecalis (17)* | AMX 25 µg | GEN 15 µg | LIN 15 µg | FUR 300 µg | VAN 30 µg | TEC 30 µg |
| *S. aureus (25)* | FOX 30 µg | CLI 2µg | SXT 1,25/23,75 µg | GEN 15 µg | VAN 30 µg | TEC 30 µg |
| *S.epidermidis (8)* | | | | | | |

Le tableau C1 liste différents phénotypes de références pour les espèces de bactéries de référence testées correspondant aux résistances ou sensibilités vis-à-vis des antibiotiques dans lequels:
- les phénotypes et codes d'antibiotiques ont les significations données précédemment,
- S signifie : sensible à l'antibiotique ou combinaison d'antibiotiques contenu dans la dite pastille, le diamètre de l'auréole d'inhibition entourant la pastille étant supérieure au diamètre correspondant à la concentration critique inférieure,
- R signifie : résistante à l'antibiotique ou combinaison d'antibiotiques contenu dans la dite pastille, le diamètre de l'auréole d'inhibition entourant la pastille étant inférieur au diamètre correspondant à la concentration critique supérieure,
- I signifie : intermédiaire par rapport à l'antibiotique ou combinaison d'antibiotiques contenu dans la dite pastille, le diamètre l'auréole d'inhibition entourant la pastille étant compris entre les valeurs de diamètres critiques correspondants aux concentration critique inférieure et concentration critique supérieure.

La dite base de donnée comprend les phénotypes de références pour les espèces de bactéries de référence correspondant à des résistances (I,R) ou sensibilités (S) suivantes vis-à-vis des concentrations des 6 composés antibiotiques mentionnés au tableau C1.;

| Bactéries | Phénotypes | Antibiotiques | | | | | |
|---|---|---|---|---|---|---|---|
| *E. coli* | | AMX | AMC | TCC | CRO | FEP | IMP |
| | Sauvage | S | S | S | S | S | S |
| | Pénicillinase de bas niveau | R | S | S | S | S | S |
| | Pénicillinase résistante aux inhibiteurs | R | R | R | S | S | S |
| | Pénicillinase de haut niveau | R | I/R | I/R | S | S | S |
| | BLSE | R | R | R | R | R | S |
| | Céphalosporinase haut niveau | R | R | R | R | R | S |
| | Carbapénémase | R | R | R | R | R | R |
| *P. mirabilis* | | AMX | AMC | TCC | CRO | FEP | IMP |
| | Sauvage | S | S | S | S | S | S |
| | Pénicillinase de bas niveau | R | S | S | S | S | S |
| | Pénicillinase résistante aux inhibiteurs | R | R | R | S | S | S |
| | Pénicillinase de haut niveau | R | I | I | S | S | S |
| | Céphalosporinase haut niveau | R | R | R | R | R | S |
| *K. pneumoniae* | | AMC | TZP | CRO | FEP | IMP | COL |
| | Sauvage | S | S | S | S | S | S |
| | Pénicillinase de bas niveau | S | R | S | S | S | S |
| | Pénicillinase de haut niveau | I/R | S/I/R | I | S | S | S |
| | BLSE | R | R/I | I/R | R | S | S |
| | Céphalosporinase haut niveau | I/R | I/R | I/R | S | S | S |
| | Carbapénémase | R | R | R | R | R | S |
| *K. oxytoca* | | AMC | TZP | CRO | FEP | IMP | COL |
| | Sauvage | S | S | S | S | S | S |
| *E. faecalis* | | AMX | GEN | LIN | FUR | VAN | TEC |
| | Sauvage | S | S | S | S | S | S |
| | Atypique | R | S | S | S | S | S |
| *E. aerogenes* | | AMC | TZP | CRO | FEP | IMP | COL |
| | Sauvage | S | S | S | S | S | S |
| | BLSE | R | R | R | R/I | S | S |
| *E. cloacae* | | AMC | TZP | CRO | FEP | IMP | COL |
| | Sauvage | S | S | S | S | S | S |
| | BLSE | R | R | R | R | S | S |
| *P. aeruginosa* | | TIC | TCC | TZP | FEP | CAZ | IMP |
| | Sauvage | S | S | S | S | S | S |
| | Perméabilité sélective à l'imipenème | S | S | S | S | S | I |
| | Pénicillinase | R | I/R | I/S | S | S | S |
| | BLSE | I/R | I/R | I/R | I/R | R | S |
| | Carbapénémase | R | R | R | R | R | R |
| | Phénotype anormal | R | R | R/S | R | S | S |
| *S. aureus* | | FOX | CLI | SXT | GEN | VAN | TEC |
| | Sauvage | S | S | S | S | S | S |
| | Résistant méthicilline | R | R/S | S | S | S | S |
| *S*. *epidermidis* | | FOX | CLI | SXT | GEN | VAN | TEC |
| | Sauvage | S | S | S | S | S | S |
| | Résistant méthicilline | R | R/S | S | S | S | S |

Les pastilles du tableau C1 ci-dessus sont disposées successivement en cercle sur les antibiogrammes dans l'ordre des colonnes de gauche à droite du tableau correspondant à l'ordre des aiguilles d'une montre sur le cercle.

Le phénotype BLSE inclut un phénotype de synergie entre la ceftriaxone et l'acide clavulanique donc entre CRO et TCC ou AMC (pour toutes les bactéries BLSE du tableau).

2.3) Les inventeurs ont vérifié que le logiciel pouvait aussi reconnaître des antibiogrammes réalisés sur boites carrés avec un plus grand nombre d'antibiotiques. Ils ont réalisés 25 antibiogrammes de 16 pastilles comprenant : AMX de 25µg; AMC de 20-10ug; GM ou GEN de 15 µg ; SXT de 1.25/23.75µg ; TIC de 75µg ; TCC de 75/10µg ; IMP ou IPM de 10µg ; COL de 50µg; CRO de 30µg; CIP de 5µg ; OFX de 5µg ; CTX de 5µg ; AN ou AK de 30µg ; TOB de 10µg ; FOX de 30µg ; ATM de 30µg, CLI ou DA 2µg, 30µg, 30µg,VA ou Van30µg, FF de 200µg, E de 15µg, NT ou FUR 300µg, PT 15µg, TEC 30 µg, LNZ 10µg.

La dite base de donnée comprend les phénotypes de références suivants pour les espèces de bactéries de référence suivantes correspondant aux résistances (I/R)/ ou sensibilités (S) suivantes vis-à-vis des 16 antibiotiques mentionnés dans le tableau C2 suivant dont les pastilles sont disposées en quadrillage dans l'ordre suivant à partir de la première rangée à droite avec 4 rangées de 4 composés alignées en colonnes pour chaque groupe:

**Tableau C2 :**

| Bactéries | Phénotypes | Antibiotiques | | | | | | | |
|---|---|---|---|---|---|---|---|---|---|
| *E. coli* | Sauvage | CIP | OFX | TIC | COL | IPM | CTX | TCC | AK |
| | | S | S | S | S | S | S | S | S |
| | | SXT | AMC | CRO | TOB | AX | ATM | FOX | GEN |
| | | S | S | S | S | S | S | S | S |
| | Pénicillinase de haut niveau + R aux Fluoroquinolones + R au Cotrimoxazole + R partielle aux aminosides | CIP | OFX | TIC | COL | IPM | CTX | TCC | AK |
| | | R | R | R | S | S | S | I | S |
| | | SXT | AMC | CRO | TOB | AX | ATM | FOX | GEN |
| | | R | I | S | R | R | S | S | R |
| | Céphalosporinase + R aux Fluoroquinolones + R au Cotrimoxazole + R partielle aux aminosides | CIP | OFX | TIC | COL | IPM | CTX | TCC | AK |
| | | R | R | R | S | S | R | R | S |
| | | SXT | AMC | CRO | TOB | AX | ATM | FOX | GEN |
| | | R | R | R | R | R | R | S | R |
| *K. pneumoniae* | Sauvage | CIP | OFX | TIC | COL | IPM | CTX | TCC | AK |
| | | S | S | R | S | S | R | R | R |
| | | SXT | AMC | CRO | TOB | AX | ATM | FOX | GEN |
| | | R | R | R | R | R | R | S | R |
| | Céphalosporinase + R aux Aminosides + R au Cotrimoxazole | CIP | OFX | TIC | COL | IPM | CTX | TCC | AK |
| | | R | R | R | S | S | R | R | S |
| | | SXT | AMC | CRO | TOB | AX | ATM | FOX | GEN |
| | | R | R | R | R | R | R | S | R |
| *S. aureus* | Sauvage | SXT | DA | FOX | OXA | PT | GEN | CIP | RA |
| | | S | S | S | S | S | S | S | S |
| | | TEC | VA | LNZ | FF | TOB | E | DO | NT |
| | | S | S | S | S | S | S | S | S |
| | R à la Méthicilline, R aux aminosides, Fluoroquinolones, à la Rifampicine, aux Macrolides et apparentés ... | SXT | DA | FOX | OXA | PT | GEN | CIP | RA |
| | | R | R | R | R | R | R | R | R |
| | | TEC | VA | LNZ | FF | TOB | E | DO | NT |
| | | S | S | S | R | R | R | R | R |

Ces phénotypes de références à 16 pastilles ci-dessus sont plus, particulièrement utiles pour les infections dites profondes comme par exemple les septicémies.

3) Banque d'images de référence et interprétation selon l'invention par comparaison d'images photographiques d'antibiogrammes.

3.1) Les photos sont réalisés avec un appareil de capture d'image comprenant un scanner le Scan^{®}1200 Interscience Réf 437 000 (INTERSCIENCE, France).

La prise des images est standardisée à l'aide d'un scanner haute résolution dont la boîte à photographier est placée dans ce scanner et la luminosité, la hauteur et la qualité de la photo en pixels est toujours la même. Chaque photo sera prise dans les mêmes conditions :
- Fond plat et noir
- Même lumière / même luminosité qui sont réglées automatiquement par le logiciel avec 6 combinaisons d'éclairage blanc par dessous et/ou pardessus, sur un fond noir.
- Même appareil de capture d'images : Scan^{®}1200 Interscience Réf 437 000.
- Même distance de 10cm
- Même échelle comprise entre 1,5 et 3.

Il fournit des photos de résolution d'au moins 1280 × 960 pixels.

3.2) Initialement, les inventeurs voulaient simplement standardiser le nombre d'antibiotiques limité à un nombre réduit, notamment uniquement 6 antibiotiques sur une seule boite pour les bactéries les plus fréquentes. Ceci les a amenés à tester la reproductibilité et la répétabilité de la technique sur un nombre important d'échantillons pour pouvoir l'automatiser. Pour ce faire, ils ont réalisé des photos de tous les résultats et ainsi ont constitué une banque de données avec ces images. Sur ce grand nombre de photos, ils les ont comparées entre elles et se sont aperçus fortuitement que les photos de chaque phénotype bien que distinctes semblaient pouvoir être reconnaissables entres elles. En d'autres termes, il semblait possible de reconnaître le phénotype de la bactérie en se basant sur une image générale de l'antibiogramme sans mesurer directement les diamètres d'inhibition sur l'antibiogramme et sans nécessiter de les interpréter selon les données d'interprétation de l'EUCAST.

Ils ont ensuite cherché à confirmer cette reconnaissance par le biais d'un logiciel de reconnaissance automatique d'images. Pour ce faire, Ils ont utilisé un logiciel de reconnaissance d'image XnView téléchargeable en ligne ((http://www.xnview.com/fr/, Gougelet Pierre-Emmanuel-10 rue René de Chateaubriand, La neuvilette-51100 REIMS-FRANCE). Ce logiciel permet de créer des répertoires de banques d'images puis de rechercher sur l'ordinateur sur lequel il est installé, des images similaires de manière ciblée (dans un autre répertoire) soit dans tous les disques de stockage de l'ordinateur. Ainsi le logiciel n'est pas « parasité » par des images qui n'auraient rien à voir avec la recherche en cours.

Ce logiciel indique également un pourcentage de similitude des photos par rapport aux photos de la banque d'image. Chaque photo correspond à une feuille qui a été scanné. Les feuilles ont été numérisées par le scanner pour obtenir des feuilles vertes sur un fond blanc et permettre une segmentation simple de la feuille. La couleur de l'image a ensuite été convertie en niveaux de gris. Les contours binaires de l'image sont tracés par analyse séquentielle. Si un pixel est trouvé, le logiciel recherche les limites les plus proches de ce point pour ne repérer que le contour. Seule la plus longue frontière de l'image est prise en compte, les autres frontières des deux objets ainsi que les trous étant considérés comme étant du bruit.

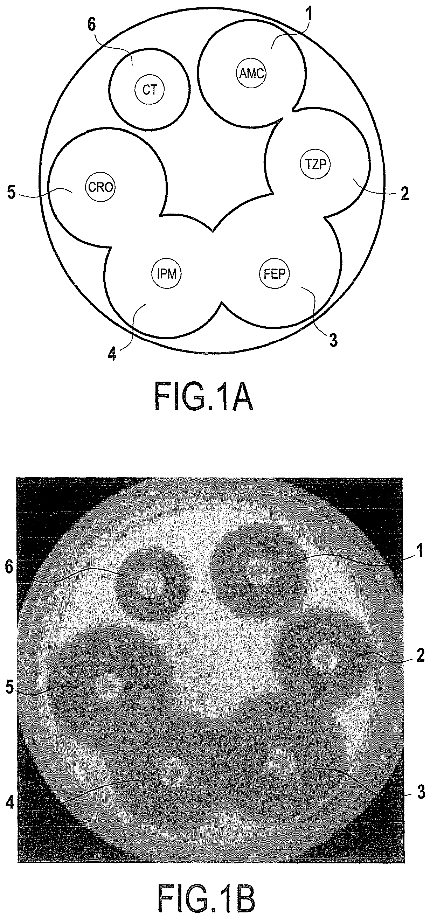

Un tel logiciel s'avère approprié pour reconnaître des photos d'antibiogrammes car seuls les diamètres d'inhibition varient d'un phénotype à l'autre ce qui se traduit par une variation du contour de l'image globale de l'antibiogramme comme montré Figures 1A et 1B. Ce logiciel est donc capable de reconnaître indirectement les différents diamètres d'inhibition et donner leurs interprétations phénotypiques par comparaison d'image globale de l'antibiogramme.

Une photo d'un antibiogramme à analyser est comparée aux images de référence de la banque d'images réalisée à partir des antibiogrammes de référence par le logiciel de reconnaissance d'image lequel peut reconnaître des photos de souches ayant une similarité ou ressemblance d'au moins 70% en pratique et ainsi donner le phénotype de référence correspondant à l'échantillon testé le cas échéant. Si l'image est reconnue par le logiciel avec une similitude d'au moins 70% par rapport à une image de référence, ceci signifie que la bactérie possède le même phénotype que l'image de référence, donc le logiciel peut lui attribuer son phénotype.

Un autre logiciel développé pour reconnaître des images comme les photos de feuilles de certaines plantes et en déduire ainsi l'espèce de la plante (Novotny P, Suk T (2013) Leaf recognition of woody species in central Europe. J Clin Microbiol), est aussi capable de reconnaître des photos d'antibiogrammes en comparaison avec des photos se trouvant dans une banque de données existante et constituée préalablement Plus particulièrement, ce logiciel est capable de reconnaître à partir des feuilles l'espèce d'un arbre à partir d'une banque de données de feuilles et plusieurs photos de feuilles pour une même espèce en comparant uniquement le contour de la feuille sans considérer tout ce qui est à l'intérieur ou à l'extérieur des contours et par analogie de forme du contour détermine l'espèce de l'arbre.

Il est possible ensuite de fournir des recommandations thérapeutiques en automatique en même temps que le phénotype lors de la validation pour que le clinicien possède l'intégralité des données.

Ainsi par exemple pour *E. coli,* au départ, les inventeurs ont créé un répertoire de photos à partir des photos d'antibiogrammes de 42 isolats cliniques d'*E*. *coli.* Dans ces 42 photos, ils en ont sélectionné plusieurs pour chaque phénotype donné. Chacune de ces photos a été renommée en fonction de son phénotype et il a été demandé au logiciel s'il était capable de reconnaître d'autres phénotypes similaires à ceux de référence et à partir de quel pourcentage de similarité le logiciel commençait à faire des erreurs au niveau de la reconnaissance des phénotypes.

En diminuant progressivement le taux de similarité de 5 en 5%, il a été déterminé un taux de similitude minimal requis de 70% avec ce logiciel pour reconnaître des photos d'antibiogrammes différentes mais avec le même phénotype sans réaliser d'erreur.

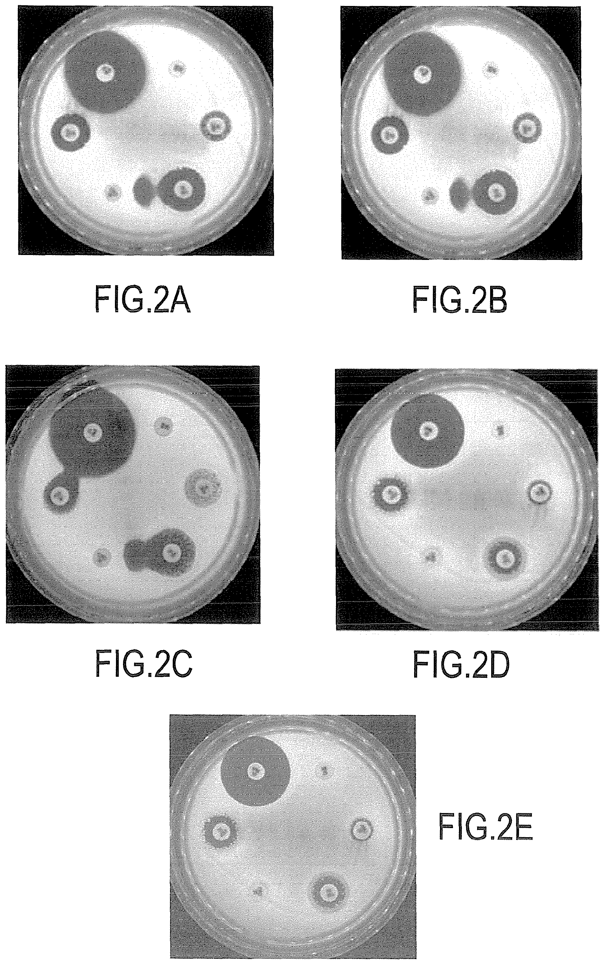

Les Figures 2A à 2C illustrent le fait que ce logiciel XnView de reconnaissance d'image est capable de donner le phénotype exact des bactéries *E coli* de phénotype BLSE avec une similarité de :
- 100% entre les images des figures 2A et 2B ;
- 87% entre les images des figures 2A et 2C.

Les Figures 2D et 2E illustrent le fait que ce logiciel XnView de reconnaissance d'image est capable de donner le phénotype exact des bactéries *E coli* de phénotype Céphalosporinase avec une similarité de :
- 74 % les images des figures 2D et 2E, et
- 65% entre les images des figures 2A et 2D se distinguant essentiellement par la présence d'une zone de synergie entre AMX et TCC pour BLSE (fig.2A à 2C) et absence de zone de synergie entre AMX et TCC pour le phenotype céphalosporinase.

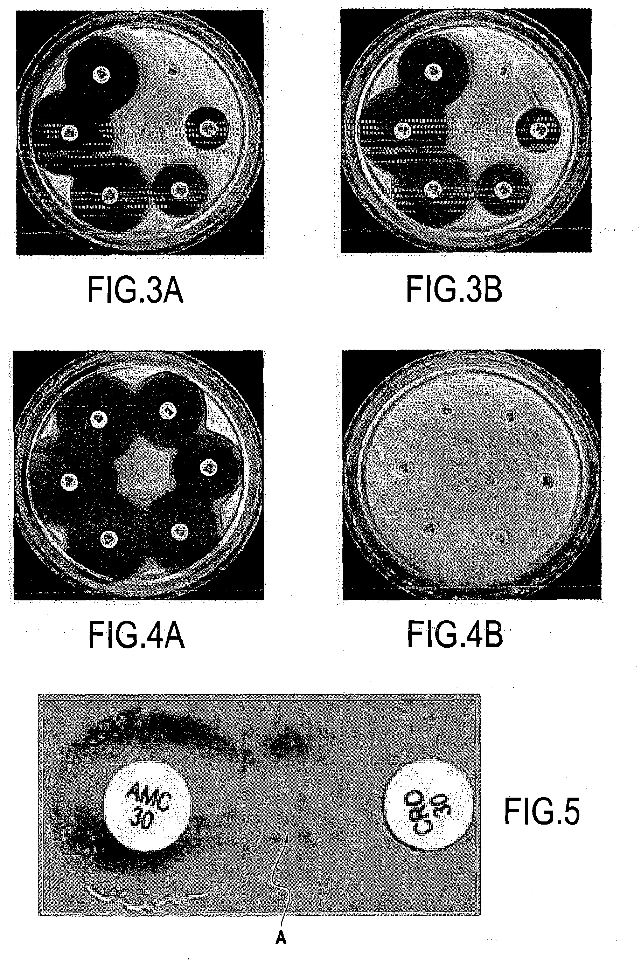

Les Figures 3A et 3B montrent que le logiciel est capable de reconnaître des photos d'antibiogrammes présentant le même phénotype « pénicillinase de haut niveau » de bactéries *E. coli* à partir d'une base de données de 42 photos de 42 échantillons de *E. coli* présentant des phénotypes variés et avec une similarité de 80 %.

Les figures 4A et 4B montrent les photos d'antibiogrammes de 2 souches *d'Escherichia coli* : celle de figure4A est sensible à tous les antibiotiques testés (souche sauvage) alors que celle de figure 4 B est résistante à tous les antibiotiques.

La figure 5-montre qu'on peut interpréter un mécanisme de résistance très particulier par des images de synergies permettant de visualiser des mécanismes de synergies entre deux antibiotiques.

Sur la figure 5, une bactérie *E. coli* ayant un mécanisme de résistance aux antibiotiques BLSE (B-Lactamase-à-spectre-élargi) présente une synergie entre deux antibiotiques particuliers la ceftriaxone (Céphalosporine de 3ième génération de la famille des b-lactamines) et l'association amoxicilline + acide clavulanique (Amoxicilline qui est une aminopénicilline + un inhibiteur de b-lactamase). Il est possible de voir cette synergie directement sur la gélose car il y a apparition d'une petite zone d'inhibition en forme dite de bouchon de champagne entre ses deux pastilles de ses antibiotiques cote à cote qui normalement sont résistants vis à vis de cette souche. La reconnaissance d'image globale d'antibiogramme permet d'identifier ce type de mécanisme de synergie entre 2 antibiotiques.

Les inventeurs ont vérifié que le logiciel pouvait aussi reconnaître des antibiogrammes réalisés sur boites carrés avec un plus grand nombre d'antibiotiques. Ils ont réalisés 25 antibiogrammes de 16 pastilles comprenant différents phénotypes décrits ci-dessus.

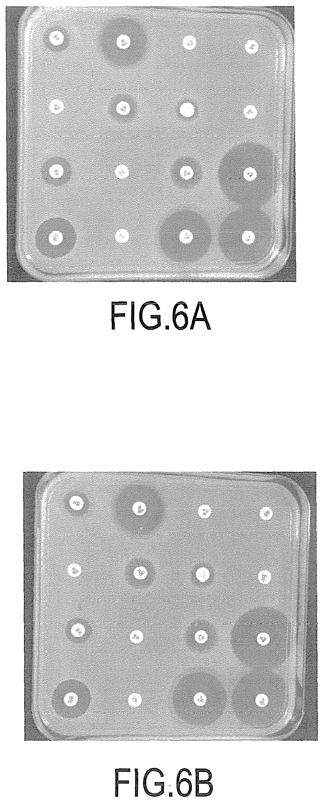

Les figures 6A et 6B montrent que le logiciel est capable de reconnaître des photos d'antibiogrammes présentant le même phénotype (« Céphalosporinase + Résistance aux aminosides et au cotrimoxazole») de bactéries *K. pneumoniae* à partir d'une base de données de 9 photos de 9 échantillons de *K. pneumoniae* présentant des phénotypes variés avec une similarité de 88%.

Le logiciel est capable de reconnaître des photos comme étant celles de même bactérie de référence ayant le même phénotype avec une similarité de 70% au minimum.

Ce taux de similitude garantit de reconnaître la couleur, les lettres des codes antibiotiques et chiffres de dosage de concentrations inscrites sur les disques, les contours de l'image des auréoles. Chaque antibiotique déposé sur la gélose possède un code à 2 ou 3 lettres permettant de l'identifier (correspondant à l'abréviation de son nom) et de vérifier que l'antibiotique présent sur la gélose est bien le bon antibiotique. Il est aussi noté le dosage de l'antibiotique. Par exemple sur le disque d'imipenème on retrouve le code « IPM 10 µg » ce qui correspond à un disque d'imipenème dosé à 10 µg. Enfin le logiciel reconnait que la disposition des pastilles en cercle ou en carrée est correcte.

Les contours et la forme de l'image sont deux choses différentes. La forme de l'image comprend la forme globale de cette image alors que les contours de l'image font ici référence qu'à une seule partie de l'image comme par exemple uniquement les contours des zones d'inhibition comme on le voir sur les figures 1A et 1B.

Compte tenu du fait que pour un même phénotype le diamètre peut varier (cf plus haut exemple entre 16 et 22 mm pour I et R si >16 ou S si > 22 mm) plus il y a d'images de référence pour un même phénotype dans la base de données meilleure est la reconnaissance d'un même phénotype même si le diamètre est légèrement différent sans changer l'interprétation.

Ce logiciel est capable à partir d'une banque de données d'images connues de chercher simultanément les images les plus similaires de façon rapide quel que soit la quantité d'images dans la banque de données. En outre, un logiciel peut générer automatiquement une interprétation complète ainsi qu'un compte rendu d'analyse et des recommandations thérapeutiques de différentes natures (commentaires ou autres selon les recommandations des comités d'experts).

L'utilisation du logiciel comprend les étapes suivantes :
a) Sélectionner un dossier contenant une photo d'antibiogramme à analyser ;
b) Sélectionner dans outils : « Rechercher des fichiers similaires » qui permet la reconnaissance d'image ;
c) sélectionner le fichier contenant la banque d'images de référence pour la comparer avec l'image à analyser ;
d) Indiquez le taux de similitude, par exemple un taux de 73% de similitude.

La comparaison est telle que chaque paire d'images obtient une valeur de similitude calculée et si cette valeur est plus grande que la tolérance minimale de 73% alors la paire est considérée comme similaire et les deux images appartiennent au même phénotype de référence.

La banque d'images de référence peut être étendue à toutes les bactéries fréquemment retrouvées chez l'homme à savoir environ 200 espèces bactériennes différentes, voire même toutes les bactéries (environ 2000 espèces isolées au moins une fois chez l'homme).

Le fait de rajouter des images de référence permet d'améliorer l'interprétation et augmenter le pourcentage seuil de similitude pour un même phénotype.

### Références bibliographiques

1) Journal of Clinical Microbiology 1998 page 302-304, Volume 36 N° 1, Kent Korgenski et al.
2) Journal of Clinical Microbiology April 2000 pages 1688-1693, volume 38 n° 4.
3) Clinical Microbiology and Infection Volume 10 N° 5 - 2004 Kolbert et al.
4) Journal of Clinical MICROBIOLOGY April 2005 pages 1846-1850 Volume 43 n° 4 - Bert et al.
5) Journal of Microbiological Methods 2008 Volume 75 pages 177-181.
6) International Journal of Antimicrobial Agents 45 (215) 61-65 - Lepage et al.
7) Salgado et al, « Automatic antibiograms inhibition halo détermination through texture and directional filtering analysis », 2001, Institute of electrical and electronics engineers, page 629-632

## Revendications

1. Méthode d'analyse et interprétation automatisée d'un antibiogramme d'un échantillon de microorganisme à analyser, de préférence une bactérie, permettant de déterminer un phénotype de résistance ou sensibilité à au moins un composé antimicrobien, ledit phénotype correspondant à la résistance ou à la sensibilité d'un échantillon de microorganisme à analyser, de préférence une bactérie, par rapport à au moins un composé antimicrobien, de préférence une pluralité de composés antimicrobiens, de préférence des composés antibiotiques, le dit phénotype étant choisi parmi une pluralité de phénotypes de référence différents pour chacune des espèces de microorganismes de référence d'une pluralité d'espèces de microorganismes de référence différentes dans laquelle:
a) l'antibiogramme de l'échantillon du microorganisme à analyser, de préférence appartenant à l'une des dites espèces de microorganismes de référence, est réalisé selon une méthodologie déterminée sur un milieu de culture solide, méthodologie comprenant:
- une concentration de microorganisme, de préférence de bactérie (UFC/mL), déterminée de l'échantillon de microorganisme, de préférence bactérie, déposée sur le dit milieu de culture solide,
- un nombre n déterminé d'une pluralité de substrats, une disposition déterminée et une forme déterminée de substrats, de préférence en forme de pastilles, contenant chacun(e) une concentration déterminée de composé(s) antimicrobien(s) déterminé(s) et aptes à diffuser un (ou des) composé(s) antimicrobien(s) déterminé(s) différente(s) pour chacun des n dits substrats déposés sur le dit milieu de culture solide, et
- des conditions et durée d'incubation déterminées de l'échantillon de microorganisme déposé sur le dit milieu de culture solide, de préférence de 18 à 24h à une température de 35-37°C, et
b) on réalise l'acquisition d'une image photographique de l'antibiogramme obtenu à l'étape a), de préférence à l'aide d'un appareil de capture d'image photographique comprenant un scanner et appareil photographique, ladite méthode étant **caractérisée en ce que** :
c) on détermine le dit phénotype de l'échantillon de microorganisme à analyser, de préférence bactérie, sans mesurer directement les diamètres d'inhibition sur l'antibiogramme, en comparant avec un logiciel de reconnaissance d'image, l'image photographique obtenue à l'étape b) avec des images de référence d'antibiogrammes de référence, d'une base de données constituée des dites images de référence, telles que la dite base de donnée comprend une pluralité d'au moins p images de référence différentes d'antibiogrammes de p souches différentes du même microorganisme, pour chaque phénotype de référence de chaque espèce de microorganisme de référence.

2. Méthode selon la revendication 1 **caractérisée en ce qu'**à l'étape c), tous les antibiogrammes de référence sont réalisés selon la même dite méthodologie que l'antibiogramme à analyser, et les dites d'images de référence et l'image à analyser sont réalisées avec le même appareil de capture d'image photographique et dans les mêmes conditions, notamment même distance, même luminosité, même fond, et même résolution.

3. Méthode selon la revendication 1 ou 2 **caractérisée en ce qu'**on met en oeuvre une pluralité de n dits substrats, n étant de 5 à 20, de préférence n= 6 à 16, et la dite base de données comprend une pluralité d'au moins p images de référence différentes d'antibiogrammes de p souches différentes du même microorganisme, p étant d'au moins égal à 5, de préférence p étant au moins égal à 10.

4. Méthode selon l'une des revendications 1 à 3 **caractérisée en ce qu'**à l'étape c), on compare avec ledit logiciel de reconnaissance d'image, la forme du contour des auréoles correspondants aux zones d'inhibition autour des substrats, de préférence des pastilles, d'antibiotiques et de préférence aussi les lettres des codes et chiffres de dosage des antibiotiques indiqués sur les pastilles d'antibiotiques.

5. Méthode selon l'une des revendications 1 à 4 **caractérisée en ce qu'**on détermine un pourcentage de similitude minimal admissible entre une image à analyser et une image de référence, dénommé ci-après seuil de similitude, de sorte que la bactérie de l'échantillon à analyser sera déterminée comme présentant un dit phénotype de référence d'une dite bactérie de référence si le logiciel de reconnaissance évalue un pourcentage de similitude entre l'image de l'antibiogramme de la bactérie de l'échantillon à analyser et une image de référence correspondant audit phénotype de référence de la dite espèce de bactérie de référence, au moins égal à un pourcentage de similitude seuil admissible minimal, de préférence un pourcentage de similitude seuil admissible minimal d'au moins 70% de préférence au moins 80%.

6. Méthode selon l'une des revendications 1 à 5 **caractérisée en ce qu'**on détermine un pourcentage de similitude seuil minimal entre les dites images de référence différentes des différents antibiogrammes des p souches différentes pour chaque phénotype de référence de chaque espèce de bactérie de référence, ci-après dénommé seuil de similitude de référence, de préférence on détermine un pourcentage de seuil de similitude de référence correspondant au pourcentage de similitude le plus bas entre les deux images de référence les plus distinctes pour chaque phénotype de référence de chaque espèce de bactérie de référence, de sorte que la bactérie de l'échantillon à analyser sera déterminée comme présentant un dit phénotype de référence d'une dite bactérie de référence si le logiciel de reconnaissance évalue un pourcentage de similitude au moins égal audit seuil de similitude de référence entre l'image de l'antibiogramme de la bactérie de l'échantillon à analyser et une image de référence correspondant audit phénotype de référence de la dite espèce de bactérie de référence.

7. Méthode selon l'une des revendications 1 à 6 **caractérisée en ce que** le dit phénotype de référence est une classification de résistance ou de sensibilité pour un antibiotique ou une association d'antibiotiques donné.

8. Méthode selon l'une des revendications 1 à 6 **caractérisée en ce que** le phénotype de référence est une classification de résistance ou sensibilité pour une pluralité d'antibiotiques et/ou d'association d'antibiotiques.

9. Méthode selon la revendication 7 **caractérisé en ce qu'**un dit phénotype de référence comprend un phénotype de synergie entre deux antibiotiques correspondant à deux antibiotiques pour lesquels la bactérie de référence est résistante lorsqu'ils sont pris séparément et pour lesquels la bactérie de référence est sensible lorsqu'ils sont pris en association.

10. Méthode selon l'une des revendications 1 à 9 **caractérisée en ce que** les dites espèces de bactéries de référence sont choisies parmi au moins *Escherichia coli, Klebsiella pneumoniae, Proteus mirabilis, Klebsiella oxytoca, Enterobacter cloacae, Enterobacter aerogenes, Enterococcus faecalis, Pseudomonas aeruginosa, Staphylococcus aureus et Staphylococcus epidermidis.*

11. Méthode selon l'une des revendications 1 à 10 **caractérisée en ce que** les antibiogrammes sont réalisés à partir de pastilles comprenant les antibiotiques suivants et avec les marquages suivants correspondants aux dits antibiotiques: AMX pour amoxicilline; AMC pour Amoxicilline-acide clavulanique; GEN pour gentamicine; SXT pour Triméthoprime-sulfamethoxazole (Cotrimoxazole); TIC, pour Ticarcilline;TCC pour Ticarcilline-acide clavulanique; TZP pour Piperacilline-tazobactam; CAZ pour Ceftazidime; IMP pour Imipenème; COL ou CL pour Colistine; FOX pour Céfoxitine; VAN ou VA pour Vancomycine; TEC pour Téicoplanine; CLI ou DA pour Clindamycine; CRO pour Ceftriaxone; FEP pour Céfépime; L ou LIN pour Lincomycine; FUR ou NT pour Nitrofurantoïne, CIP pour Ciprofloxacine; OFX pour l'Ofloxacine; CTX pour Céfotaxime; AK ou AN pour Amikacine; TOB ou TM pour Tobramycine; FOX pour Céfoxitine; ATM pour Aztréonam; ERT pour Ertapénème; FF ou FOS pour Fosfomycine; DO pour Doxycycline; K pour Kanamycine; L ou LIN pour Lincomycine; LNZ pour Linézolide; NA pour Acide Nalidixique; NOR pour Norfloxacine; MET pour Métronidazole; MEM pour Méropénème; PT pour Pristinamycine; P pour Pénicilline G; RA pour Rifampicine; TGC pour Tigécycline; TEL pour Télithromycine; E pour Erythromycine, OX ou OXA pour Oxacilline.

12. Méthode selon la revendication 1 à 11 **caractérisée en ce que** le phénotype de référence est une classification de résistance ou choisie parmi au moins les phénotypes suivants :
- Phénotype «sauvage»,
- Phénotype «Pénicillinase de bas niveau»,
- Phénotype dénommé «Pénicillinase résistante aux inhibiteurs »,
- Phénotype «Pénicillinase de haut niveau»,
- Phénotype «Béta-Lactamase à Spectre étendu» (BLSE),
- Phénotype «Céphalosporinase haut niveau,
- Phénotype «Carbapénèmase»,
- Phénotype «Perméabilité sélective à l'imipenème»,
- Phénotype «Résistance à la méthicilline»,
- Phénotype «résistance aux fluoroquinolones»,
- Phénotype « résistance aux aminosides»,
- Phénotype «résistance aux macrolides»,
- Phénotype «résistance au Cotrimaxole»,
- Phénotype «résistance à la Rifampicine»,
- Phénotype dénommé «Phénotype atypique».

13. Méthode selon la revendication 11 **caractérisée en ce que** l'antibiogramme analysé et/ou la dite base de donnée comprend les phénotypes de références pour les espèces de bactéries de référence correspondant à des résistances (I,R) ou sensibilités (S) suivantes vis-à-vis des concentrations des 6 composés antibiotiques choisis parmi les groupes a1) à a6) suivants, de préférence disposés en cercle dans l'ordre suivant pour chaque groupe:
a1) AMX, AMC, TCC, CRO, FEP et IMP,
a2) AMX, AMC, TZP, CRO, FEP et IMP,
a3) AMC, TZP, CRO, FEP, IMP et COL,
a4) AMX, GEN, LIN,FUR, VAN et TEC,
a5) TIC, TCC, TZP, FEP, CAZ et IMP,
a6) FOX, CLI, SXT, GEN, VAN et TEC.

14. Méthode selon la revendication 11 **caractérisée en ce que** l'antibiogramme analysé et/ou la dite base de donnée comprend les phénotypes de références suivants pour les espèces de bactéries de référence suivantes correspondant aux résistances (I/R)/ ou sensibilités (S) suivantes vis-à-vis des 16 composés antibiotiques choisis parmi les groupes b1) et b2) suivants, de préférence dont les pastilles sont disposées en quadrillage dans l'ordre suivant à partir de la première rangée à droite avec 4 rangées de 4 composés alignées en colonnes pour chaque groupe :
b1) CIP,OFX, TIC, COL, IMP, CTX, TCC, AKSXT, AMC, CRO, TOB, AMX, ATM, FOX et GEN,
b2) SXT, DA, FOX, OXA, PT, GEN, CIP, RA, TEC, VAN, LNZ, TOB, E, DO et NT.

15. Méthode selon l'une des revendications 1 à 14 **caractérisée en ce qu'**à l'étape a), on réalise les antibiogrammes de référence et de l'échantillon de bactérie à analyser, selon une méthodologie déterminée sur un milieu de culture solide de type gélose de Muller-Hinton, comprenant:
- un nombre (n), de 6 à 16 pastilles en forme de disques, à des concentrations en antibiotique de 10 à 300ug, disposées en cercle ou en quadrillage,
- une concentration bactérienne (UFC/mL) déterminée de l'échantillon à analyser ou de référence déposée sur le dit milieu de culture solide, correspondant à une turbidité de la suspension déposée correspondant à l'étalon de 0.5 dans la gamme de McFarland, et
- des conditions et durée d'incubation déterminée de 18 à 24h à 37°C.

## Patentansprüche

1. Verfahren zur automatisierten Analyse und Interpretation eines Antibiogramms einer zu analysierenden Mikroorganismusprobe, vorzugsweise eines Bakteriums, das ermöglicht, einen Phänotyp der Resistenz oder Empfindlichkeit gegenüber mindestens einer antimikrobiellen Verbindung zu bestimmen, wobei der Phänotyp der Resistenz oder der Empfindlichkeit einer zu analysierenden Mikroorganismusprobe, vorzugsweise eines Bakteriums, in Bezug auf mindestens eine antimikrobielle Verbindung, vorzugsweise mehrere antimikrobielle Verbindungen, vorzugsweise antibiotische Verbindungen entspricht, wobei der Phänotyp aus mehreren verschiedenen Referenzphänotypen für jede der Referenzmikroorganismusarten aus mehreren verschiedenen Referenzmikroorganismusarten ausgewählt ist, wobei:
a) das Antibiogramm der zu analysierenden Mikroorganismusprobe, die vorzugsweise zu einer der Referenzmikroorganismusarten gehört, gemäß einer bestimmten Methodik auf einem festen Kulturmedium umgesetzt wird, wobei die Methodik umfasst:
- eine Mikroorganismuskonzentration, vorzugsweise Bakterien (UFC/ml), bestimmt aus der Mikroorganismusprobe, vorzugsweise Bakterien, die auf dem festen Kulturmedium abgelagert ist,
- eine bestimmte Anzahl n aus mehreren Substraten, eine bestimmte Anordnung und eine bestimmte Form von Substraten, vorzugsweise in Form von Tabletten, die jeweils eine bestimmte Konzentration von/einer bestimmten antimikrobiellen Verbindung(en) enthalten und in der Lage sind, eine (oder mehrere) verschiedene bestimmte antimikrobielle Verbindung(en) für jedes der n Substrate, die auf dem festen Kulturmedium abgelagert sind, zu verbreiten, und
- bestimmte Inkubationsbedingungen und -dauer der Mikroorganismusprobe, die auf dem festen Kulturmedium abgelagert ist, vorzugsweise 18 bis 24 h bei einer Temperatur von 35-37 °C, und
b) die Erfassung eines fotografischen Bildes des Antibiogramms, das in Schritt a) erhalten wird, umgesetzt wird, vorzugsweise mit der Hilfe eines fotografischen Bilderfassungsgeräts, das einen Scanner und einen Fotoapparat umfasst, wobei das Verfahren **dadurch gekennzeichnet ist, dass**:
c) der Phänotyp der zu analysierenden Mikroorganismusprobe, vorzugsweise Bakterien, bestimmt wird, ohne die Hemmdurchmesser auf dem Antibiogramm direkt zu messen, indem mit einer Bilderkennungssoftware das fotografische Bild, das in Schritt b) erhalten wird, mit Referenzbildern von Referenzantibiogrammen aus einer Datenbank, die aus den Referenzbildern besteht, verglichen wird, sodass die Datenbank mehrere mindestens p verschiedene Referenzbilder von Antibiogrammen von p verschiedenen Stämmen des gleichen Mikroorganismus für jeden Referenzphänotyp jeder Referenzmikroorganismusart umfasst.

2. Verfahren nach Anspruch 1, **dadurch gekennzeichnet, dass** in Schritt c) alle Referenzantibiogramme gemäß der gleichen Methodik wie das zu analysierende Antibiogramm umgesetzt werden und die Referenzbilder und das zu analysierende Bild mit dem gleichen fotografischen Bilderfassungsgerät und unter den gleichen Bedingungen, insbesondere dem gleichen Abstand, der gleichen Helligkeit, dem gleichen Hintergrund und der gleichen Auflösung umgesetzt werden.

3. Verfahren nach Anspruch 1 oder 2, **dadurch gekennzeichnet, dass** mehrere n Substrate eingesetzt werden, wobei n von 5 bis 20 ist, vorzugsweise n = 6 bis 16, und die Datenbank mehrere mindestens p verschiedene Referenzbilder von Antibiogrammen von p verschiedenen Stämmen des gleichen Mikroorganismus umfasst, wobei p mindestens gleich 5 ist, vorzugsweise p mindestens gleich 10 ist.

4. Verfahren nach einem der Ansprüche 1 bis 3, **dadurch gekennzeichnet, dass** in Schritt c) die Form der Kontur der Halos, die den Hemmzonen um die Substrate entsprechen, vorzugsweise Tabletten, Antibiotika und vorzugsweise auch den Buchstaben von Dosierungscodes und -nummern der Antibiotika, die auf den Antibiotikatabletten angegeben sind, mit der Bilderkennungssoftware verglichen wird.

5. Verfahren nach einem der Ansprüche 1 bis 4, **dadurch gekennzeichnet, dass** ein minimal zulässiger Ähnlichkeitsprozentsatz zwischen einem zu analysierenden Bild und einem Referenzbild bestimmt wird, im Folgenden Ähnlichkeitsschwelle genannt, sodass bestimmt wird, dass das Bakterium der zu analysierenden Probe einen Referenzphänotyp eines Referenzbakteriums aufweist, wenn die Erkennungssoftware einen Ähnlichkeitsprozentsatz zwischen dem Bild des Antibiogramms des Bakteriums der zu analysierenden Probe und einem Referenzbild, das dem Referenzphänotyp der Referenzbakterienart entspricht, mindestens gleich einem minimal zulässigen Ähnlichkeitsschwellenprozentsatz, vorzugsweise einem minimal zulässigen Ähnlichkeitsschwellenprozentsatz von mindestens 70 %, vorzugsweise mindestens 80 % bewertet.

6. Verfahren nach einem der Ansprüche 1 bis 5, **dadurch gekennzeichnet, dass** ein minimaler Ähnlichkeitsschwellenprozentsatz zwischen den verschiedenen Referenzbildern der verschiedenen Antibiogramme der p verschiedenen Stämme für jeden Referenzphänotyp jeder Art von Referenzbakterien bestimmt wird, im Folgenden Referenzähnlichkeitsschwelle genannt, vorzugsweise ein Referenzähnlichkeitsschwellenprozentsatz, der dem niedrigsten Ähnlichkeitsprozentsatz zwischen den zwei unterschiedlichsten Referenzbildern für jeden Referenzphänotyp jeder Referenzbakterienart entspricht, sodass bestimmt wird, dass das Bakterium der zu analysierenden Probe einen Referenzphänotyp eines Referenzbakteriums aufweist, wenn die Erkennungssoftware einen Ähnlichkeitsprozentsatz mindestens gleich der Referenzähnlichkeitsschwelle zwischen dem Bild des Antibiogramms des Bakteriums der zu analysierenden Probe und einem Referenzbild, das dem Referenzphänotyp der Referenzbakterienart entspricht, bewertet.

7. Verfahren nach einem der Ansprüche 1 bis 6, **dadurch gekennzeichnet, dass** der Referenzphänotyp eine Klassifikation der Resistenz oder Empfindlichkeit gegenüber einem gegebenen Antibiotikum oder einer Kombination von Antibiotika ist.

8. Verfahren nach einem der Ansprüche 1 bis 6, **dadurch gekennzeichnet, dass** der Referenzphänotyp eine Klassifikation der Resistenz oder Empfindlichkeit gegenüber mehreren Antibiotika und/oder einer Kombination von Antibiotika ist.

9. Verfahren nach Anspruch 7, **dadurch gekennzeichnet, dass** der Referenzphänotyp einen Synergiephänotyp zwischen zwei Antibiotika umfasst, die zwei Antibiotika entsprechen, gegenüber denen das Referenzbakterium resistent ist, wenn sie separat genommen werden, und gegenüber denen das Referenzbakterium empfindlich ist, wenn sie in Kombination genommen werden.

10. Verfahren nach einem der Ansprüche 1 bis 9, **dadurch gekennzeichnet, dass** die Referenzbakterienarten mindestens aus *Escherichia coli, Klebsiella pneumoniae, Proteus mirabilis, Klebsiella oxytoca, Enterobacter cloacae, Enterobacter aerogenes, Enterococcus faecalis, Pseudomonas aeruginosa, Staphylococcus aureus* und *Staphylococcus epidermidis* ausgewählt sind.

11. Verfahren nach einem der Ansprüche 1 bis 10, **dadurch gekennzeichnet, dass** die Antibiogramme aus Tabletten, welche die folgenden Antibiotika umfassen und mit den folgenden Markierungen, die den Antibiotika entsprechen, umgesetzt sind: AMX für Amoxicillin, AMC für Amoxicillin-Clavulansäure, GEN für Gentamicin, SXT für Trimethoprim-Sulfamethoxazol (Cotrimoxazol), TIC für Ticarcillin, TCC für Ticarcillin-Clavulansäure, TZP für Piperacillin-Tazobactam, CAZ für Ceftazidim, IMP für Imipenem, COL oder CL für Colistin, FOX für Cefoxitin, VAN oder VA für Vancomycin, TEC für Teicoplanin, CLI oder DA für Clindamycin, CRO für Ceftriaxon, FEP für Cefepim, L oder LIN für Lincomycin, FUR oder NT für Nitrofurantoin, CIP für Ciprofloxacin, OFX für Ofloxacin, CTX für Cefotaxim, AK oder AN für Amikacin, TOB oder TM für Tobramycin, FOX für Cefoxitin, ATM für Aztreonam, ERT für Ertapenem, FF oder FOS für Fosfomycin, DO für Doxycyclin, K für Kanamycin, L oder LIN für Lincomycin, LNZ für Linezolid, NA für Nalidixinsäure, NOR für Norfloxacin, MET für Metronidazol, MEM für Meropenem, PT für Pristinamycin, P für Penicillin G, RA für Rifampicin, TGC für Tigecyclin, TEL für Telithromycin, E für Erythromycin, OX oder OXA für Oxacillin.

12. Verfahren nach Anspruch 1 bis 11, **dadurch gekennzeichnet, dass** der Referenzphänotyp eine Resistenzklassifikation oder aus mindestens den folgenden Phänotypen ausgewählt ist:
- "wilder" Phänotyp,
- Phänotyp "Penicillinase mit niedrigem Niveau",
- Phänotyp namens "Inhibitor-resistente Penicillinase",
- Phänotyp "Penicillinase mit hohem Niveau",
- Phänotyp "Beta-Lactamase mit erweitertem Spektrum" (BLSE),
- Phänotyp "Cephalosporinase hohes Niveau",
- Phänotyp "Carbapenemase",
- Phänotyp "selektive Permeabilität für Imipenem",
- Phänotyp "Resistenz gegenüber Methicillin",
- Phänotyp "Resistenz gegenüber Fluorchinolonen",
- Phänotyp "Resistenz gegenüber Aminoglykosiden",
- Phänotyp "Resistenz gegenüber Makroliden",
- Phänotyp "Resistenz gegenüber Cotrimaxol",
- Phänotyp "Resistenz gegenüber Rifampicin",
- Phänotyp namens "atypischer Phänotyp".

13. Verfahren nach Anspruch 11, **dadurch gekennzeichnet, dass** das analysierte Antibiogramm und/oder die Datenbank die Referenzphänotypen für die Referenzbakterienarten umfasst/en, die den folgenden Resistenzen (I, R) oder Empfindlichkeiten (S) gegenüber Konzentrationen von 6 antibiotischen Verbindungen ausgewählt aus den folgenden Gruppen a1) bis a6) entsprechen, vorzugsweise kreisförmig in der folgenden Reihenfolge für jede Gruppe angeordnet:
a1) AMX, AMC, TCC, CRO, FEP und IMP,
a2) AMX, AMC, TZP, CRO, FEP und IMP,
a3) AMC, TZP, CRO, FEP, IMP und COL,
a4) AMX, GEN, LIN, FUR, VAN und TEC,
a5) TIC, TCC, TZP, FEP, CAZ und IMP,
a6) FOX, CLI, SXT, GEN, VAN und TEC.

14. Verfahren nach Anspruch 11, **dadurch gekennzeichnet, dass** das analysierte Antibiogramm und/oder die Datenbank die folgenden Referenzphänotypen für die folgenden Referenzbakterienarten umfasst/en, die den folgenden Resistenzen (I/R)/ oder Empfindlichkeiten (S) gegenüber 16 antibiotischen Verbindungen ausgewählt aus den folgenden Gruppen b1) und b2) entsprechen, deren Tabletten vorzugsweise in einem Gitter in der folgenden Reihenfolge angeordnet sind, beginnend mit der ersten Reihe rechts mit 4 Reihen von 4 Verbindungen, die in Spalten für jede Gruppe ausgerichtet sind:
b1) CIP, OFX, TIC, COL, IMP, CTX, TCC, AKSXT, AMC, CRO, TOB, AMX, ATM, FOX und GEN,
b2) SXT, DA, FOX, OXA, PT, GEN, CIP, RA, TEC, VAN, LNZ, TOB, E, DO und NT.

15. Verfahren nach einem der Ansprüche 1 bis 14, **dadurch gekennzeichnet, dass** in Schritt a) die Referenzantibiogramme und die zu analysierende Bakterienprobe gemäß einer Methodik umgesetzt werden, die auf einem festen Kulturmedium vom Typ Müller-Hinton-Agar bestimmt ist, umfassend:
- eine Anzahl (n) von 6 bis 16 Tabletten in Form von Scheiben mit Antibiotikakonzentrationen von 10 bis 300 µg, kreisförmig oder gitterförmig angeordnet,
- eine bestimmte Bakterienkonzentration (UFC/ml) der zu analysierenden Probe oder der Referenz, die auf dem festen Kulturmedium abgelagert ist, entsprechend einer Trübung der abgelagerten Suspension entsprechend dem Standard von 0,5 in dem McFarland-Bereich, und
- bestimmte Inkubationsbedingungen und -dauer von 18 bis 24 h bei 37 °C.

## Claims

1. A method of automatically analyzing and interpreting an antibiogram of a sample of a microorganism for analysis, preferably a bacterium, the method serving to determine a phenotype of resistance or sensitivity to at least one antimicrobial compound, preferably to a plurality of antimicrobial compounds, preferably antibiotic compounds, said phenotype being selected from a plurality of different reference phenotypes for each of the reference species of microorganism in a plurality of different species of reference microorganisms, wherein:
a) the antibiogram of the sample of the microorganism for analysis, preferably belonging to one of said species of reference microorganisms, is prepared using a determined methodology on a solid culture medium, which methodology comprises:
▪ a determined concentration of microorganism, preferably of bacterium (CFU/mL), of the sample of microorganism, preferably of bacterium, deposited on said solid culture medium;
▪ a determined number n of a plurality of substrates deposited on said solid culture medium, the substrates being in a determined arrangement and of a determined form, preferably in the form of wafers, each containing a determined concentration of determined antimicrobial compound(s) and suitable for diffusing one or more different determined antimicrobial compounds for each of the n said substrates; and
▪ determined conditions and determined duration of incubation of the sample of microorganism deposited on said solid culture medium under, preferably 18 h to 24 h at a temperature in the range 35°C to 37°C; and
b) acquiring a photographic image of the antibiogram obtained in step a), preferably using a photographic image capture apparatus comprising a scanner and camera; and
c) determining said phenotype of the sample of microorganism for analysis, preferably of bacterium, by using image recognition software to compare the photographic image obtained in step b) with reference images of reference antibiograms in a database made up of said reference images, such that said database comprises a plurality of at least p different reference images of antibiograms of p different strains of the same microorganism for each reference phenotype of each reference species of microorganism.

2. A method according to claim 1, **characterized in that** in step c), all of the reference antibiograms are prepared using the same said methodology as the antibiogram for analysis, and said reference images and the image for analysis are taken with the same photographic image capture apparatus and under the same conditions, in particular the same distance, the same brightness, the same background, and the same resolution.

3. A method according to claim 1 or claim 2, **characterized in that** a plurality, n, of said substrates is used, where n lies in the range 5 to 20, preferably n = 6 to 16, and said database comprises a plurality of at least p different reference images of antibiograms of p different strains of the same microorganism, where p is not less than 5, with p preferably being not less than 10.

4. A method according to any one of claims 1 to 3, **characterized in that** in step c), said image recognition software is used to compare the shape of the outline of the rings corresponding to the inhibition zones around the substrates, preferably antibiotic wafers, and preferably also the antibiotic code letters and dosage numerals specified on the antibiotic wafers.

5. A method according to any one of claims 1 to 4, **characterized in that** a minimum acceptable similarity percentage between an image for analysis and a reference image is determined, referred to below as a similarity threshold, such that the bacterium of the sample for analysis is determined as presenting a said reference phenotype of a said reference bacterium if the recognition software evaluates a similarity percentage between the image of the antibiogram of the bacterium of the sample for analysis and a reference image corresponding to said reference phenotype of said reference species of bacterium that is not less than a minimum acceptable threshold similarity percentage, preferably a minimum acceptable threshold similarity percentage of at least 70%, more preferably at least 80%.

6. A method according to any one of claims 1 to 5, **characterized in that** a minimum threshold similarity percentage between said different reference images of different antibiograms of p different strains for each reference phenotype of each reference species of bacterium is determined, referred below as the reference similarity threshold, and preferably a reference similarity threshold percentage corresponding to the lowest similarity percentage between the two most different reference images for each reference phenotype of each reference species of bacteria is determined, such that the bacterium of the sample for analysis is determined as presenting a said reference phenotype of a said reference bacterium if the recognition software evaluates a similarity percentage that is not less than said reference similarity threshold between the image of the antibiogram of the bacterium of the sample for analysis and a reference image corresponding to said reference phenotype of said reference species of bacterium.

7. A method according to any one of claims 1 to 6, **characterized in that** said reference phenotype is a resistance or sensitivity classification for a given antibiotic or association of antibiotics.

8. A method according to any one of claims 1 to 6, **characterized in that** the reference phenotype is a resistance or sensitivity classification for a plurality of antibiotics and/or of associations of antibiotics.

9. A method according to claim 7, **characterized in that** a said reference phenotype includes a synergy phenotype between two antibiotics corresponding to two antibiotics for which the reference bacterium is resistant when they are taken separately and for which the reference bacterium is sensitive when they are taken in association.

10. A method according to any one of claims 1 to 9, **characterized in that** said reference species of bacteria are selected from at least *Escherichia coli, Klebsiella pneumoniae, Proteus mirabilis, Klebsiella oxytoca, Enterobacter cloacea, Enterobacter aerogenes, Entercoccus faecalis, Pseudomonas aeruginosa, Staphylococcus aureus,* and *Staphylococcus epidermidis.*

11. A method according to any one of claims 1 to 10, **characterized in that** the antibiograms are made using wafers comprising the following antibiotics and with the following marking corresponding to said antibiotics: AMX for amoxicillin; AMC for amoxicillin-clavulanic acid; GEN for gentamicin; SXT for trimethoprim-sulfamethoxazole (cotrimoxazole); TIC for ticarcillin; TCC for ticarcillin-clavulanic acid; TZP for piperacillin-tazobactam; CAZ for ceftazidime; IMP for imipenem; COL or CL for colistin; FOX for cefoxitine; VAN or VA for vancomycin; TEC for teicoplanin; CLI or DA for clindamycin; CRO for ceftriaxone; FEP for cefepime; L or LIN for lincomycin; FUR or NT for nitrofurantoin; CIP for ciprofloxacin; OFX for ofloxacin; CTX for cefotaxime; AK or AN for amikacin; TOB or TM for tobramycin; FOX for cefoxitine; ATM for aztreonam; ERT for ertapenem; FF or FOS for fosfomycin; DOI for doxycycline; K for kanamycin; L or LIN for lincomycin; LNZ for linezolid; NA for nalidixic acid; NOR for norfloxacin; MET for metronidazole; MEM for meropenem; PT for pristinamycin; P for penicillin G; RA for rifampicin; TGC for tigecycline; TEL for telithromycin; E for erythromycin; and OX or OXA for oxacillin.

12. A method according to any one of claims 1 to 11, **characterized in that** the reference phenotype is a resistance classification or selected from among at least the following phenotypes:
▪ phenotype "wild",
▪ phenotype "low level penicillinase",
▪ phenotype referred to as "penicillinase resistant to inhibitors",
▪ phenotype "high level penicillinase",
▪ phenotype "broad spectrum beta-lactamase" (BSBL),
▪ phenotype "high level cephalosporinase",
▪ phenotype "carbapenemase",
▪ phenotype "selective permeability to imipenem",
▪ phenotype "resistance to methicillin",
▪ phenotype "resistance to fluoroquinolones",
▪ phenotype "resistance to aminosides",
▪ phenotype "resistance to macrolides",
▪ phenotype "resistance to cotrimoxazole",
▪ phenotype "resistance to rifampicin", and
▪ phenotype known as "atypical phenotype".

13. A method according to claim 11, **characterized in that** the antibiogram under analysis and/or said database comprises the reference phenotypes for the reference species of bacteria corresponding to the following resistances (I, R) or sensitivities (S) relative to concentrations of six antibiotic compounds selected from the following groups a1) to a6), preferably arranged in a circle in the following order for each group:
a1) AMX, AMC, TCC, CRO, FEP, and IMP,
a2) AMX, AMC, TZP, CRO, FEP, and IMP,
a3) AMC, TZP, CRO, FEP, IMP, and COL,
a4) AMX, GEN, LIN, FUR, VAN, and TEC,
a5) TIC, TCC, TZP, FEP, CAZ, and IMP, and
a6) FOX, CLI, SXT, GEN, VAN, and TEC.

14. A method according to claim 11, **characterized in that** the antibiogram under analysis and/or said database comprises the following reference phenotypes for the following reference species of bacteria corresponding to the following resistances (I/R) or sensitivities (S) with respect to sixteen antibiotic compounds selected from the following groups b1) and b2), preferably having their wafers arranged in a rectangular grid in the following order starting from the first row on the right with four rows of four compounds aligned in columns for each group:
b1) CIP, OFX, TIC, COL, IMP, CTX, TCC, AK, SXT, AMC, CRO, TOB, AMX, ATM, FOX, and GEN, and
b2) SXT, DA, FOX, OXA, PT, GEN, CIP, RA, TEC, VAN, LNZ, TOB, E, DO, and NT.

15. A method according to any one of claims 1 to 14, **characterized in that** in step a), the reference antibiograms and the antibiogram of the sample of bacterium for analysis are prepared using a determined methodology on a solid culture medium of Mueller-Hinton agar type comprising:
▪ a number (n) of six to sixteen wafers in the form of disks having antibiotic concentrations in the range 10 µg to 300 µg, arranged in a circle or a rectangular grid;
▪ a determined bacterial concentration (CFU/mL) of the sample for analysis or of the reference deposited on said solid culture medium, corresponding to a deposited suspension having opacity matching the 0.5 standard in the McFarland range; and
▪ determined incubation conditions and duration of 18 h to 24 h at 37°C.
